(19) Europäisches Patentamt
European Patent Office
Office européen des brevets

(11) EP 4 775 598 A1

(12) EUROPEAN PATENT APPLICATION
published in accordance with Art. 153(4) EPC

(43) Date of publication:
**15.07.2026 Bulletin 2026/29**

(21) Application number: **24850479.7**

(22) Date of filing: **07.03.2024**

(51) International Patent Classification (IPC):
***C08B 37/00*** $^{(2006.01)}$ ***A61K 31/715*** $^{(2006.01)}$
***A61P 35/00*** $^{(2006.01)}$

(52) Cooperative Patent Classification (CPC):
**A61P 35/00; A61K 31/716; A61K 33/243; C08B 37/0003; C08B 37/0024**

(86) International application number:
**PCT/CN2024/080445**

(87) International publication number:
**WO 2025/030819 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.08.2023 CN 202310982992**

(71) Applicant: **Shenzhen Yandai Investment Co., Ltd**
**Shenzhen, Guangdong 518000 (CN)**

(72) Inventors:
- **CHEN, Xuemin**
  **Shenzhen, Guangdong 518000 (CN)**
- **MO, Guan**
  **Shenzhen, Guangdong 518000 (CN)**
- **HE, Chunming**
  **Shenzhen, Guangdong 518000 (CN)**
- **CHEN, Jin**
  **Shenzhen, Guangdong 518000 (CN)**
- **LIU, Yunlei**
  **Shenzhen, Guangdong 518000 (CN)**
- **LI, Yanjun**
  **Shenzhen, Guangdong 518000 (CN)**
- **CHEN, Yao**
  **Shenzhen, Guangdong 518000 (CN)**
- **CHEN, Fengji**
  **Shenzhen, Guangdong 518000 (CN)**
- **LIANG, Mei**
  **Shenzhen, Guangdong 518000 (CN)**
- **FANG, Zhe**
  **Shenzhen, Guangdong 518000 (CN)**

(74) Representative: **Metida**
**Gyneju str. 16**
**01109 Vilnius (LT)**

# (54) POLYSACCHARIDE COMPOUND HAVING CLEAR MOLECULAR STRUCTURE AND BEING CAPABLE OF ELIMINATING TOXIC SIDE EFFECTS OF CHEMOTHERAPEUTIC DRUGS

(57) This invention pertains to the field of plant extraction and separation technology, and provides a polysaccharide compound with a defined molecular structure that possesses antitumor efficacy and mitigates the toxic side effects of chemotherapy. The polysaccharide compound is intended for the treatment of advanced cancer patients (those who are no longer candidates for surgery, have a life expectancy of only three to six months, and are still eligible for chemotherapy). The molecular formula of the polysaccharide compound is: $(C_{66}H_{110}O_{55})_n$. The active ingredient, polysaccharide, is obtained through the extraction of effective components from Ganoderma lucidum under high temperature and high pressure. The polysaccharide with this active ingredient, characterized by its water solubility, is readily absorbed by the human body, offers antitumor effects, and has proven efficacy in the prevention of tumor development in humans. Notably, when used in combination with chemotherapy drugs, the polysaccharide can alleviate toxic side effects caused by the chemotherapy drugs on the human body and has been supported by experimental data showing control and reduction of tumor masses, and reduction and elimination of cancer cells.

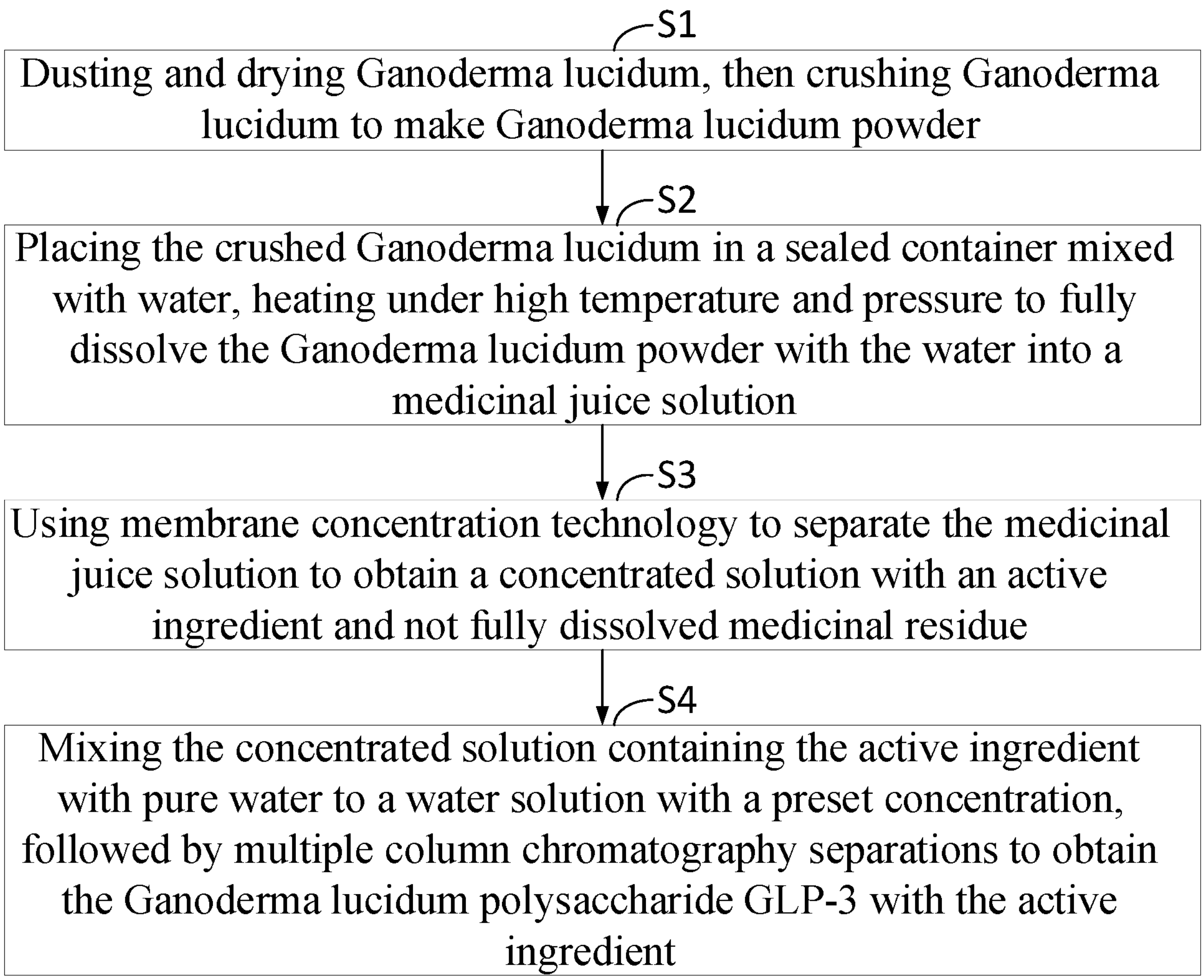
S1
Dusting and drying Ganoderma lucidum, then crushing Ganoderma lucidum to make Ganoderma lucidum powder
S2
Placing the crushed Ganoderma lucidum in a sealed container mixed with water, heating under high temperature and pressure to fully dissolve the Ganoderma lucidum powder with the water into a medicinal juice solution
S3
Using membrane concentration technology to separate the medicinal juice solution to obtain a concentrated solution with an active ingredient and not fully dissolved medicinal residue
S4
Mixing the concentrated solution containing the active ingredient with pure water to a water solution with a preset concentration, followed by multiple column chromatography separations to obtain the Ganoderma lucidum polysaccharide GLP-3 with the active ingredient

Fig. 1

## Description

### Technical Field

The present invention relates to the field of plant extraction and separation technology improvements, and particularly relates to a method and usage of extracting a polysaccharide compound, i.e., Ganoderma lucidum polysaccharide GLP-3, with a defined molecular structure, which has antitumor efficacy and can eliminate the toxic side effects of chemotherapy drugs.

### Background

Ganoderma lucidum is a type of fungal plant with a long history of medicinal use in China and Japan. Ganoderma lucidum has a plurality of complex active ingredients, and over 150 compounds have been isolated from it, such as polysaccharides, triterpenes, sterols, alkaloids, furan derivatives, amino peptides, and inorganic elements. Geographic location (longitude and latitude), seed variation, growth environment, and differences in temperature, humidity, and light intensity can significantly affect the content, proportion, and presence of an active ingredient referred to in this invention in Ganoderma lucidum.

### Summary of Invention

This invention provides a polysaccharide compound with a defined molecular structure that has antitumor efficacy and can eliminate the toxic side effects of chemotherapy drugs.

This invention aims to address the unmet international challenges of treating patients with advanced cancer, where "patients with advanced cancer" are defined as: those who are no longer surgical candidates, have a life expectancy of only three to six months, and are still eligible for chemotherapy; the "challenges of treating patients" refer to: allowing patients with advanced cancer to regain their appetite quickly (typically within two to three weeks), reducing or stabilizing tumor size, and using in combination with chemotherapy drugs to essentially eliminate the toxic side effects induced by the chemotherapy drugs on the human body. Long-term combination use with the chemotherapy drugs can achieve the objective of substantially eradicating cancer cells or keeping the number of cancer cells within safe limits for high-quality human survival.

Another important function of the active ingredients specified in this invention is the prevention of mutations in normal human cells and the prevention of cancer cell formation.

Furthermore, the active ingredient in this invention, when used in combination with the chemotherapy drugs, exhibit exceptionally good therapeutic effects on lung cancer, liver cancer, and breast cancer, demonstrating a certain degree of broad-spectrum activity.

The invention provides a method for extracting Ganoderma lucidum polysaccharide GLP-3, comprising the steps of:

S1. dusting and drying Ganoderma lucidum, then crushing Ganoderma lucidum to make Ganoderma lucidum powder;
S2. placing the crushed Ganoderma lucidum in a sealed container mixed with water, heating under high temperature and pressure to fully dissolve the Ganoderma lucidum powder with the water into a medicinal juice solution;
S3. using membrane concentration technology to separate the medicinal juice solution to obtain a concentrated solution with an active ingredient and not fully dissolved medicinal residue; and
S4. mixing the concentrated solution containing the active ingredient with pure water to a water solution with a preset concentration, followed by multiple column chromatography separations as well as concentration and lyophilization to obtain the Ganoderma lucidum polysaccharide GLP-3 with the active ingredient.

A further aspect of the invention: In step S2, the Ganoderma lucidum powder is thoroughly mixed with the water and heated to 105-200°C, and the boiling time lasts for 2-6 h, during which the Ganoderma lucidum powder and the water in the sealed container are fully dissolved under high temperature and pressure into a mixed medicinal liquid.

A further aspect of the invention: In step S2, the Ganoderma lucidum powder is thoroughly mixed with the water and heated to 105-170°C, and the boiling time lasts for 3-6 h, during which the Ganoderma lucidum powder and the water in the sealed container are fully dissolved under high temperature and pressure into a mixed medicinal liquid.

A further aspect of the invention: In step S4, the concentrated liquid containing the active ingredient is mixed with the pure water at a concentration ratio of 1:2 to 1:5.

A further aspect of the invention: In step S3, Ganoderma lucidum residue is removed from the extracted water solution containing the active ingredient by using the membrane concentration technology, obtaining a concentrated liquid

or paste containing the active ingredient.

A further aspect of the invention: In step S1, the Ganoderma lucidum is rinsed with clean water to remove surface dust, then dried at 105°C in drying equipment, and after drying, the Ganoderma lucidum is crushed in crushing equipment to obtain the Ganoderma lucidum powder, with a particle size larger than 60 mesh.

A further aspect of the invention: In step S2, the mixed liquid in the sealed container is heated to temperatures of 105°C, 110°C, 115°C, 120°C, 125°C, 130°C, 135°C, 140°C, 145°C, 150°C, 155°C, 160°C, 165°C, or 170°C, with boiling times of 3 h, 3.5 h, 4 h, 4.5 h, 5 h, 5.5 h, or 6 h, during which the Ganoderma lucidum powder in the sealed container is fully dissolved with the water into the mixed medicinal liquid in the high temperature and pressure environment.

The invention also provides the Ganoderma lucidum polysaccharide GLP-3, wherein the structural formula of the Ganoderma lucidum polysaccharide GLP-3 is

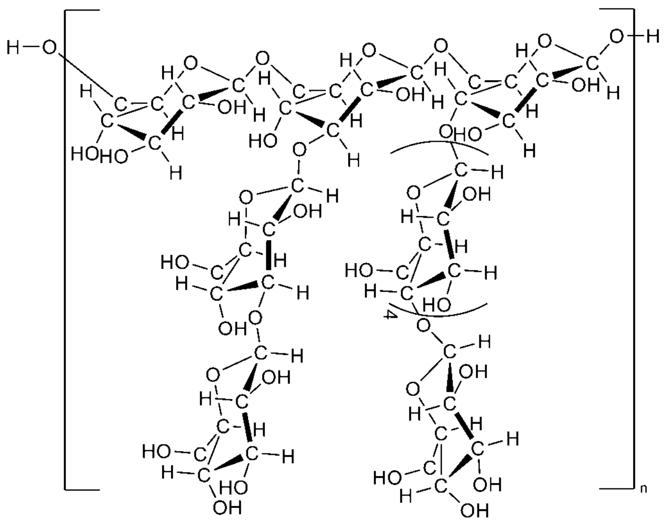

; the molecular formula is $(C_{66}H_{110}O_{55})_n$, where n = 30-46.

A further aspect of the invention: n is 30, 31, 32, 33, 34, 35, 36, 37, 38, 39, 40, 41, 42, 43, 44, 45, or 46.

This invention also provides the application of the Ganoderma lucidum polysaccharide GLP-3. The Ganoderma lucidum polysaccharide GLP-3 is characterized by its water solubility, is readily absorbed by the human body, offers antitumor effects, and has proven strong efficacy in the prevention of tumor development in humans. Notably, when used in combination with chemotherapy drugs, it can alleviate toxic side effects caused by the chemotherapy drugs on the human body, control and reduce tumor masses, and reduce and eliminate cancer cells.

The beneficial effects of this invention include a simple extraction process, high polysaccharide yield, low production cost, and ease of operation. The resultant Ganoderma lucidum polysaccharide is highly soluble and readily absorbed by the human body, enhancing its antitumor effects.

Brief Description of the Drawings

Figure 1: Flowchart of the method for extracting Ganoderma lucidum polysaccharide GLP-3, provided by an embodiment of the invention.

Figure 2: Schematic diagram of 1gMp-RT (peak molecular weight) calibration curves provided by an embodiment of the invention.

Figure 3: Schematic diagram of 1gMp-RT (weight-average molecular weight) calibration curvew provided by an embodiment of the invention.

Figure 4: Schematic diagram of 1gMp-RT (number-average molecular weight) calibration curves provided by an embodiment of the invention.

Figure 5: Schematic diagram of the molecular weight of Ganoderma lucidum polysaccharide GLP-3, provided by an embodiment of the invention.

Figure 6: Schematic diagram of the polysaccharide infrared spectrum provided by an embodiment of the invention.

Figure 7: Schematic diagram 1 of the ion chromatography of mixed standard 16 sugars, provided by an embodiment of the invention.

Figure 8: Schematic diagram 2 of the ion chromatography of mixed standard 16 sugars, provided by an embodiment of the invention.

Figure 9: GCMS chromatogram of the sample (PMAA) provided by an embodiment of the invention.

Figure 10: Schematic diagram 1 of the analysis results of permethylated alditol acetate (PMAA) of polysaccharide, provided by an embodiment of the invention.

Figure 11: Schematic diagram 2 of the analysis results of permethylated alditol acetate (PMAA) of polysaccharide, provided by an embodiment of the invention.

Figure 12: Schematic diagram 3 of the analysis results of permethylated alditol acetate (PMAA) of polysaccharide, provided by an embodiment of the invention.

Figure 13: Schematic diagram 4 of the analysis results of permethylated alditol acetate (PMAA) of polysaccharide, provided by an embodiment of the invention.

Figure 14: Schematic diagram 5 of the analysis results of permethylated alditol acetate (PMAA) of polysaccharide, provided by an embodiment of the invention.

Figure 15: Schematic diagram 6 of the analysis results of permethylated alditol acetate (PMAA) of polysaccharide, provided by an embodiment of the invention.

Figure 16: Schematic diagram of the hydrogen spectrum provided by an embodiment of the invention.

Figure 17: Schematic diagram of the carbon spectrum provided by an embodiment of the invention.

Figure 18: Schematic diagram of the Dept135 spectrum provided by an embodiment of the invention.

Figure 19: Schematic diagram of HH-COSY provided by an embodiment of the invention.

Figure 20: Schematic diagram of HSQC provided by an embodiment of the invention.

Figure 21: HMBC spectrum provided by an embodiment of the invention.

Figure 22: Schematic diagram of NOESY provided by an embodiment of the invention.

Figure 23: Schematic diagram of the control group of LLC-bearing mouse model treated with Ganoderma lucidum polysaccharide GLP-3 combined with chemotherapy, provided by an embodiment of the invention.

Figure 24: Schematic diagram of the cisplatin group of LLC-bearing mice treated with Ganoderma lucidum polysaccharide GLP-3 combined with chemotherapy, provided by an embodiment of the invention.

Figure 25: Schematic diagram of the cisplatin + low-dose GLP-3 group of LLC-bearing mice treated with Ganoderma lucidum polysaccharide GLP-3 combined with chemotherapy, provided by an embodiment of the invention.

Figure 26: Schematic diagram of the cisplatin + high-dose GLP-3 group of LLC-bearing mice treated with Ganoderma lucidum polysaccharide GLP-3 combined with chemotherapy, provided by an embodiment of the invention.

Figure 27: Schematic diagram of tumor models in the control group of LLC-bearing mice treated with Ganoderma lucidum polysaccharide GLP-3 combined with chemotherapy, provided by an embodiment of the invention.

Figure 28: Schematic diagram of tumors in the cisplatin group of LLC-bearing mice treated with Ganoderma lucidum

polysaccharide GLP-3 combined with chemotherapy, provided by an embodiment of the invention.

Figure 29: Schematic diagram of tumors in the cisplatin + low-dose GLP-3 group of LLC-bearing mice treated with Ganoderma lucidum polysaccharide GLP-3 combined with chemotherapy, provided by an embodiment of the invention.

Figure 30: Schematic diagram of tumors in the cisplatin + high-dose GLP-3 group of LLC-bearing mice treated with Ganoderma lucidum polysaccharide GLP-3 combined with chemotherapy, provided by an embodiment of the invention.

Figure 31: Schematic diagram of the control group of H22-bearing mouse model treated with Ganoderma lucidum polysaccharide GLP-3 combined with chemotherapy, provided by an embodiment of the invention.

Figure 32: Schematic diagram of the cisplatin group of H22-bearing mice treated with Ganoderma lucidum polysaccharide GLP-3 combined with chemotherapy, provided by an embodiment of the invention.

Figure 33: Schematic diagram of the cisplatin + low-dose GLP-3 group of H22-bearing mice treated with Ganoderma lucidum polysaccharide GLP-3 combined with chemotherapy, provided by an embodiment of the invention.

Figure 34: Schematic diagram of the cisplatin + high-dose GLP-3 group of H22-bearing mice treated with Ganoderma lucidum polysaccharide GLP-3 combined with chemotherapy, provided by an embodiment of the invention.

Figure 35: External appearance schematic diagram of tumor models in the control group of H22-bearing mice treated with Ganoderma lucidum polysaccharide GLP-3 combined with chemotherapy, provided by an embodiment of the invention.

Figure 36: External appearance schematic diagram of tumors in the cisplatin group of H22-bearing mice treated with Ganoderma lucidum polysaccharide GLP-3 combined with chemotherapy, provided by an embodiment of the invention.

Figure 37: External appearance schematic diagram of tumors in the cisplatin + low-dose GLP-3 group of H22-bearing mice treated with Ganoderma lucidum polysaccharide GLP-3 combined with chemotherapy, provided by an embodiment of the invention.

Figure 38: External appearance schematic diagram of tumors in the cisplatin + high-dose GLP-3 group of H22-bearing mice treated with Ganoderma lucidum polysaccharide GLP-3 combined with chemotherapy, provided by an embodiment of the invention.

Figure 39: Schematic diagram of the effect of Ganoderma lucidum polysaccharide GLP-3 combined with radiotherapy on the tumor of orthotopic H22-bearing mice (binning: $8 \times 8$; T = 30 s), provided by an embodiment of the invention.

Figure 40: Schematic diagram of the effect of Ganoderma lucidum polysaccharide GLP-3 combined with radiotherapy on the tumor of orthotopic H22-bearing mice, provided by an embodiment of the invention.

Figure 41: Schematic diagram of the effect of Ganoderma lucidum polysaccharide GLP-3 combined with radiotherapy on the liver of orthotopic H22-bearing mice ($\times$200), provided by an embodiment of the invention.

Figure 42: Schematic diagram of the effect of Ganoderma lucidum polysaccharide GLP-3 combined with radiotherapy on the spleen of orthotopic H22-bearing mice ($\times$200), provided by an embodiment of the invention.

Figure 43: Schematic diagram of the effect of Ganoderma lucidum polysaccharide GLP-3 combined with radiotherapy on the thymus of orthotopic H22-bearing mice ($\times$200), provided by an embodiment of the invention.

## Detailed Description of the Embodiments

Embodiments of the present invention are described in detail below, and examples of the embodiments are shown in the drawings, wherein the same or similar reference numerals denote the same or similar elements or elements having the same or similar functions throughout. The embodiments below described by reference to the drawings are

exemplary and are intended for the purpose of explaining the invention and should not be construed as limiting the scope of the invention.

As shown in Figure 1, the present invention provides a flowchart for the method for extracting the Ganoderma lucidum polysaccharide GLP-3, described in detail as follows:

In step S1, harvested Ganoderma lucidum, either raw or having undergone preliminary processing, is washed with clean water in washing equipment to remove surface dust. After dust removal, the Ganoderma lucidum is transferred to drying equipment for high-temperature drying at a temperature of 105°C. The dried Ganoderma lucidum is then placed in a crusher or mill to be broken down into Ganoderma lucidum powder. The crushed Ganoderma lucidum powder is sieved, and particles of the Ganoderma lucidum powder larger than 60 mesh are screened out, while particles of the Ganoderma lucidum powder smaller than 60 mesh are returned to the crusher or mill for further crushing. This process is repeated multiple times until the crushed Ganoderma lucidum powder meets specified requirements.

In step S2, the Ganoderma lucidum powder that meets the requirements is mixed with pure water and placed in a sealed container. The sealed container is heated, in which the temperature is continuously increased to create a high-temperature, high-pressure environment, facilitating the thorough dissolution of the Ganoderma lucidum powder with the water to form a mixed solution. The sealed container is heated to a temperature between 105°C and 200°C, with a boiling time of 2-6 h, preferably between 105°C and 170°C, for 3-6 h. More preferably, the heating occurs at temperatures of 105°C, 110°C, 115°C, 120°C, 125°C, 130°C, 135°C, 140°C, 145°C, 150°C, 155°C, 160°C, 165°C, 170°C, 175°C, 180°C, 185°C, 190°C, 195°C, or 200°C, with a boiling time of 2 h, 2.5 h, 3 h, 3.5 h, 4 h, 4.5 h, 5 h, 5.5 h, or 6 h, allowing the thorough dissolution. The sealed container is a reaction kettle.

In step S3, the water solution extracted, containing the active ingredient, is centrifuged to remove residues. The medicinal liquid is then concentrated using membrane concentration technology to obtain a concentrated liquid.

In step S4, the concentrated liquid containing the active ingredient is prepared at a specific concentration, separated by column chromatography, and then concentrated and lyophilized to obtain Ganoderma lucidum polysaccharide GLP-3 with the active ingredient.

This method offers a simple extraction process, high polysaccharide yield, low production costs, and simple operation.

The invention also provides the Ganoderma lucidum polysaccharide GLP-3, wherein the structural formula of the Ganoderma lucidum polysaccharide GLP-3 is

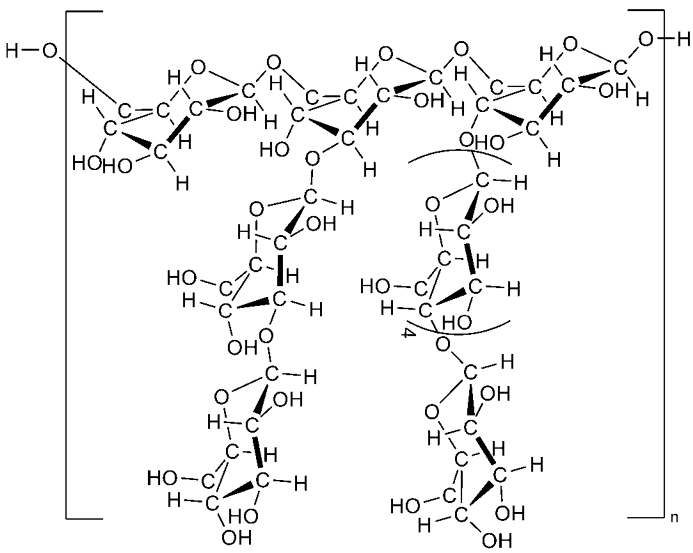

; the molecular formula is $(C_{66}H_{110}O_{55})_n$, where n = 30-46.

n is 30, 31, 32, 33, 34, 35, 36, 37, 38, 39, 40, 41, 42, 43, 44, 45, or 46.

Following the acquisition of the Ganoderma lucidum polysaccharide GLP-3 with the aforementioned structure, assay experiments were conducted and the following results are reported herein.

I. Molecular Weight Determination

1. Experimental Objective

To determine the molecular weight and purity of the polysaccharide using HPGPC.

2. Experimental Materials

2.1 Equipment

| Equipment name | Manufacturer | Model |
|---|---|---|
| High-performance liquid chromatography | Shimadzu | LC-10A |
| Differential refractometer | Shimadzu | RI-10A |
| BRT105-104-102 tandem gel permeation chromatography column | BoRui Saccharide | BRT105-104-102 (8 × 300 mm) |
| Electronic scale | Sartorius | CPA225D |
| Centrifuge | Eppendorf | Eppendorf5424 |
| Pipette | Sartorius | 200 uL, 1,000 uL |

2.2 Materials

| Reagent | Manufacturer | Batch No. | Catalog No. | Grade | Shelf life |
|---|---|---|---|---|---|
| NaCl | ACROS | A0356762 | 139725000 | ACROS | 2022 |

2.3 Standards

| Standard | Manufacturer | Batch No. | Storage conditions | Purity | Shelf life |
|---|---|---|---|---|---|
| Dextranstandards 1152 | Yuanye Bio-Technology | A16A8L41850 | Seal and store | 99% | 2 years |
| 5000 | Sigma | 102084138 | Seal and store | ≥ 99% | 2 years |
| 11600 | Sigma | 102136543 | Seal and store | 99% | 2 years |
| 23800 | Sigma | 102124529 | Seal and store | ≥ 97% | 2 years |
| 48600 | Sigma | 102104509 | Seal and store | ≥ 99% | 2 years |
| 80900 | Sigma | 102108375 | Seal and store | > 98% | 2 years |
| 148000 | Sigma | 102089360 | Seal and store | > 98% | 2 years |
| 273000 | Sigma | 102110878 | Seal and store | 98% | 2 years |
| 409800 | Sigma | 102124507 | Seal and store | > 98% | 2 years |
| 667800 | Sigma | 102104510 | Seal and store | > 98% | 2 years |
| Molecular weight is measured in Daltons (Da). | | | | | |

3. Experimental Procedure

3.1 Reagent Preparation

| Reagent name | Preparation method | Storage conditions | Shelf life |
|---|---|---|---|
| 0.05M NaCl solution | Precisely prepared, filtered through a 0.45-μm membrane, degassed by sonication for 10 min | RT | 1 month |

3.2 Preparation of Sample and Standard Solutions

Samples and standards were precisely weighed to prepare a 5 mg/mL solution, which was centrifuged at 12,000 rpm for 10 min, and the supernatant was filtered through a 0.22-μm micropore filter. Then the sample was transferred to a 1.8-mL sample vial.

3.3 Chromatographic Method

Column: BRT105-104-102 tandem gel permeation chromatography column (8 × 300 mm); Mobile phase: 0.05M NaCl solution; Flow rate: 0.6 mL/min; Column temperature: 40°C; Injection volume: 20 μL; Detector: Differential refractometer RI-10A.

4. Experimental Results

As shown in Figures 2-4, calibration curves for 1gMp-RT (peak molecular weight), 1gMw-RT (weight-average molecular weight), and 1gMn-RT (number-average molecular weight) were obtained.

The equation of the calibration curves for 1gMp-RT is: $y = -0.184x + 11.752R^2 = 0.9956$;
The equation of the calibration curves for 1gMw-RT is: $y = -0.1961x + 12.315R^2 = 0.9934$;
The equation of the calibration curves for 1gMn-RT is: $y = -0.1818x + 11.589R^2 = 0.992$;
Using the standard curves, a formula was derived to calculate the molecular weight of each sample. The molecular weight chromatograms for the samples are shown in Figure 5, with the results detailed in the table below.

| Sample ID | RT (min) | 1gMp | 1gMw | 1gMn | Mp | Mw | Mn | Peak Area Ratio (%) |
|---|---|---|---|---|---|---|---|---|
| | 37.747 | 4.8 | 4.9 | 4.7 | 64055 | 81811 | 53284 | 100 |
| Note: The peak at 46.2 min corresponds to the mobile phase. | | | | | | | | |

II. Monosaccharide Composition Determination Experiment

1. Experimental Objective

To determine the monosaccharide composition using an ion chromatograph.

2. Experimental Principle

This method is based on the electrochemical activity of sugar molecules and their ionization in strong alkaline solutions. Sugar compounds are weak acids with a pKa greater than 11. In high pH eluents, they partially or fully exist as anions. Efficient anion exchange and separation of sugar compounds is achieved, which leverages differences in ion exchange due to variations in pKa of different sugars, as well as differences in hydrophobic interactions between some sugars and the anion exchange resin. Detection is then accomplished by measuring the current produced by the oxidation of hydroxyl groups in the sugar molecules at a gold electrode surface.

3. Experimental Materials

3.1 Equipment

| Equipment name | Manufacturer | Model |
|---|---|---|
| Ion chromatograph | ThermoFisher | ICS5000 |
| Electric thermostatic blast drying oven | LICHEN | 101-1BS |
| Nitrogen blower | LICHEN | UGC-24M |
| Electronic scale | Sartorius | BS 210 S |
| Centrifuge | ThermoFisher | D-37520 |
| Pipette | DRAGONLAB | 19050983 |

3.2 Reagents

| Reagent | Manufacturer | Batch No. | Catalog No. | Grade |
|---|---|---|---|---|
| Trifluoroacetic acid | ACROS | A0356762 | 139725000 | AR |
| 50% sodium hydroxide solution | Alfa Aesar | Z21E036 | 33382 | GR |
| Sodium acetate | ThermoFishe | 191126 | 059326 | GR |

3.3 Standards

| Standard | Manufacturer | Batch No. | Storage conditions | Purity |
|---|---|---|---|---|
| Mannose | Bo Rui Saccharide | C17D9H77586 | Seal and store | AR |
| Rhamnose | Bo Rui Saccharide | H10S9Z69863 | Seal and store | AR |
| Galacturonic acid | Bo Rui Saccharide | K02A9B66077 | Seal and store | AR |
| Galactose | Bo Rui Saccharide | E1927035 | Seal and store | AR |
| Glucose | Bo Rui Saccharide | Q18F10N80946 | Seal and store | AR |
| Glucuronic acid | Bo Rui Saccharide | K14M10S82777 | Seal and store | AR |
| Arabinose | Bo Rui Saccharide | S15A10G85850 | Seal and store | AR |
| Xylose | Bo Rui Saccharide | A22S6X3606 | Seal and store | AR |
| Fucose | Bo Rui Saccharide | X29D7Y27768 | Seal and store | AR |
| Glucosamine hydrochloride | Bo Rui Saccharide | A22S6X3606 | Seal and store | AR |
| N-acetyl-D-glucosamine | Bo Rui Saccharide | A21J8X40372 | Seal and store | AR |
| D-fructose | Bo Rui Saccharide | J01J10R89818 | Seal and store | AR |
| D-ribose | Bo Rui Saccharide | H26F10Z81556 | Seal and store | AR |
| Galactosamine hydrochloride | Bo Rui Saccharide | B01J8S37079 | Seal and store | AR |
| L-guluronic acid | Bo Rui Saccharide | S200115AG1 | Seal and store | ≥98% |
| D-mannuronic acid | Bo Rui Saccharide | S200108AM1 | Seal and store | ≥98% |

4. Experimental Methods

4.1 Reagent Preparation

| Reagent name | Preparation method | Storage conditions |
|---|---|---|
| 15 mM NaOH solution | 2.4 g of 50% NaOH solution, 2 L of water | RT |
| 15 mM NaOH & 100 mM NaOAc solution | 1.2 g of 50% NaOH solution, 8.2 g of NaOAc, 1 L of water | RT |

4.2 Preparation and Calculation Method for Standard Solutions

Standard stock solutions were prepared using 16 different monosaccharide standards (fucose, rhamnose, arabinose, galactose, glucose, xylose, mannose, fructose, ribose, galacturonic acid, glucuronic acid, glucosamine hydrochloride, galactosamine hydrochloride, N-acetyl-D-glucosamine, guluronic acid, and mannuronic acid).

Concentration standards of each monosaccharide standard solution were accurately prepared and used as a mixed standard. The mass of different monosaccharides was determined using an absolute quantification method and the molar ratios were calculated based on the molar mass of each monosaccharide.

4.3 Sample Preparation

5 mg of the sample was accurately weighed in an ampule. 2 mL of 3M TFA was added and hydrolyzed at 120°C for 3 h. The acid hydrolysate was accurately transferred to a tube and evaporated to dryness under nitrogen. 5 mL water was added, vortexed to mix, and then 50 μL was taken and added to 950 μL of deionized water, and centrifuged at 12,000 rpm for 5 min. The supernatant was transferred for IC analysis.

4.4 Chromatographic Method

Column: Dionex Carbopac™ PA20 (3 × 150 mm); Mobile phase: A: $H_2O$; B: 15 mM NaOH; C: 15 mM NaOH & 100 mM NaOAc; Flow rate: 0.3 mL/min; Injection volume: 5 μL; Column temperature: 30°C; Detector: Electrochemical detector.

4.5 Standard Series

| No. | Name | ppm | Name | RT | Area |
|---|---|---|---|---|---|
| 1 | Fucose | 5 | Fuc | 5.659 | 18.741 |
| 2 | Galactosamine hydrochloride | 3 | GalN | 10.084 | 23.888 |
| 3 | Rhamnose | 5 | Rha | 10.475 | 10.717 |
| 4 | Arabinose | 3.7 | Ara | 11.092 | 16.035 |
| 5 | Glucosamine hydrochloride | 5 | GlcN | 12.367 | 31.057 |
| 6 | Galactose | 5 | Gal | 13.767 | 17.597 |
| 7 | Glucose | 5 | Glc | 15.484 | 20.442 |
| 8 | N-acetyl-D-glucosamine | 5 | GlcNAc | 16.792 | 13.652 |
| 9 | Xylose | 5 | Xyl | 17.834 | 22.737 |
| 10 | Mannose | 5 | Man | 18.117 | 14.734 |
| 11 | Fructose | 15 | Fru | 20.534 | 12.857 |
| 12 | Ribose | 10 | Rib | 22.484 | 26.868 |
| 13 | Galacturonic acid | 5 | GalA | 45.125 | 8.815 |
| 14 | Guluronic acid | 10 | GulA | 45.950 | 20.824 |

(continued)

| No. | Name | ppm | Name | RT | Area |
|---|---|---|---|---|---|
| 15 | Glucuronic acid | 5 | GlcA | 48.509 | 11.689 |
| 16 | Mannuronic acid | 10 | ManA | 50.992 | 22.847 |
| C (standard) / A (standard) = C (sample) / A (sample) | | | | | |

5. Experimental Results

Mixed standard: Solvent peaks at 2.0 min for sodium hydroxide and at 41 min for sodium acetate, as shown in Figures 6 and 7.

| Name | RT | Molar Ratio |
|---|---|---|
| Fucose | 5.659 | 0.000 |
| Galactosamine hydrochloride | 10.084 | 0.000 |
| Rhamnose | 10.475 | 0.000 |
| Arabinose | 11.092 | 0.000 |
| Glucosamine hydrochloride | 12.367 | 0.000 |
| Galactose | 13.767 | 0.000 |
| Glucose | 15.475 | 1.000 |
| N-acetyl-D-glucosamine | 16.792 | 0.000 |
| Xylose | 17.834 | 0.000 |
| Mannose | 18.117 | 0.000 |
| Fructose | 20.534 | 0.000 |
| Ribose | 22.484 | 0.000 |
| Galacturonic acid | 45.125 | 0.000 |
| Guluronic acid | 45.95 | 0.000 |
| Glucuronic acid | 48.509 | 0.000 |
| Mannuronic acid | 50.992 | 0.000 |

III. Experiment on the Determination of Polysaccharide Linkage

1. Experimental Objective

To determine the linkage patterns of polysaccharide samples through derivatization such as methylation by GC-MS analysis.

2. Experimental Materials

2.1 Equipment

| Equipment name | Manufacturer | Model |
|---|---|---|
| Rotary evaporator | Zhengzhou Greatwall Scientific Industrial and Trade Co., Ltd. | R-1001VN |
| Nitrogen blower | LICHEN | UGC-24M |

(continued)

| Equipment name | Manufacturer | Model |
|---|---|---|
| Magnetic stirrer | DLAB | MS7-H550-Pro |
| Vacuum drying oven | LICHEN | 101-1BS |
| Gas chromatograph-mass spectrometer | Agilent | 6890-5973 |

2.2 Reagents

| Reagent | Manufacturer | Batch No. | Catalog No. | Grade |
|---|---|---|---|---|
| Trifluoroacetic acid | ACROS | A0356762 | 139725000 | AR |
| Methyl iodide | Adamas | P1345479 | 01111630 | AR |
| Sodium borohydride | Aldrich | MKCD7945 | 205591 | AR |
| Ethyl acetate | Vokai | 08050003 | 40065982 | AR |
| Acetic anhydride | HUSHI | 20170314 | 10000318 | AR |
| Perchloric acid | Aldrich | SHBF7833V | 311421 | AR |
| Acetic acid | Fisher | 156174 | A35-500 | AR |
| Methanol | Merck | 10941735810 | 67-56-1 | AR |
| Sodium hydroxide | HUSHI | 20150429 | 10019718 | AR |
| Dimethyl sulfoxide | Adamas | P1265087 | 759270 | AR |
| Sodium hydride | Adamas | P1306059 | 81778A | AR |
| Methylation kit | Borui Saccharide | BRT-2020JJH | BRT-JJH | AR |

3. Experimental Methods

3.1 Reagent Preparation

| Reagent name | Preparation method | Storage conditions |
|---|---|---|
| 3M trifluoroacetic acid | 1V trifluoroacetic acid + 3V water | Store in refrigerator at 5°C |
| Sodium hydride dry powder | 60% sodium hydride washed with hexane | Store dry at room temperature |
| Sodium borodeuteride and sodium hydroxide solution | 20 mg + 20 mM NaOH solution | Seal and store |
| 20% acetic acid methanol solution | 1V glacial acetic acid + 4V water | Store in refrigerator at 5°C |
| Polysaccharide methylation kit | A: anhydrous alkaline solution | Store in refrigerator at 5°C |
| | B: methyl iodide solution | |

3.2 Sample Methylation

The sample underwent methylation, hydrolysis, and acetylation, followed by GC-MS analysis, which was compared with the standard mass spectral library.

2-3 mg of the polysaccharide sample was weighed and placed in a glass reaction vial, 1 mL of anhydrous DMSO

was added, and methylation reagent A was added rapidly. The solution was sealed and dissolved under ultrasonication, then methylation reagent B was added. The reaction was allowed at 30°C in a magnetic stirring water bath for 60 min. Finally, 2 mL of ultrapure water was added to stop the methylation reaction.

The methylated polysaccharide was taken, 1 mL of 2M trifluoroacetic acid (TFA) was added, and hydrolyzed for 90 min. The rotary evaporator was used to dry. 2 mL of double-distilled water was added to the residue, which was reduced with 60 mg of sodium borohydride for 8 h, neutralized with glacial acetic acid, and evaporated using the rotary evaporator. Then, it was dried in a 101°C oven, then 1 mL of acetic anhydride was added for acetylation and reacted at 100°C for 1 h, and cooled. Then 3 mL of toluene was added, vacuum concentrated to dry, and this process was repeated 4-5 times to remove excess acetic anhydride.

The acetylated product was dissolved in 3 mL of $CH_2Cl_2$ and transferred to a separatory funnel. A small amount of distilled water was added and shaken thoroughly, then the upper aqueous layer was removed. This process was repeated four times. The $CH_2Cl_2$ layer was dried with an adequate amount of anhydrous sodium sulfate, brought to a volume of 10 mL, and placed in a vial for liquid analysis. The acetylated sample was analyzed using a Shimadzu GCMS-QP 2010 gas chromatograph-mass spectrometer;

GC-MS Conditions: RXI-5 SIL MS column 30 m × 0.25 mm × 0.25 μm; Temperature program: started at 120°C, increased at 3°C/min to 250°C, held for 5 min; Injector temperature at 250°C, detector temperature at 250°C, carrier gas was helium, flow rate was 1 mL/min.

4. Experimental Results

The GC-MS chromatogram of the sample (PMAA) is shown in Figure 9.

The permethylated alditol acetate (PMAA) analysis of the polysaccharide is presented in the following table and Figures 10-15.

| RT | Methylated sugar | Mass fragments (m/z) | Molar ratio | Type of linkage |
|---|---|---|---|---|
| 17. 12 | 2, 3, 4, 6-$Me_4$-Glcp | 45, 71, 87, 101, 117, 129, 145, 161, 205 | 0. 184 | Glcp-(1→ |
| 21. 056 | 2, 4, 6-$Me_3$-Glcp | 45, 60, 71, 85, 101, 117, 129, 161, 189, 233 | 0. 121 | →3)-Glcp-(1→ |
| 21. 564 | 2, 3, 6-$Me_3$-Glcp | 45, 87, 99, 101, 113, 117, 129, 161, 189, 233 | 0. 324 | →4)-Glcp-(1→ |
| 22. 319 | 2, 3, 4-$Me_3$-Glcp | 45, 71, 87, 99, 101, 117, 129, 161, 189, 233 | 0. 193 | →6-Glcp-(1→ |
| 26. 871 | 2, 3-$Me_2$-Glcp | 45, 74, 85, 87, 99, 101, 117, 127, 159, 161, 201 | 0. 089 | →4, 6)-Glcp-(1→ |
| 27. 167 | 2, 4-$Me_2$-Glcp | 45, 87, 117, 129, 159, 189, 233 | 0. 088 | →3, 6)-Glcp-(1→ |

IV. NMR Spectral Analysis and Interpretation

1. Experimental Materials and Equipment

Deuterium oxide ($D_2O$, 99.9%) and deuterated acetone as internal standard; freeze-dryer, Bruker 600M Nuclear Magnetic Resonance (NMR) spectrometer;

2. Experimental Procedure

50 mg of the polysaccharide sample was weighed and dissolved in 0.5 mL of deuterium oxide followed by freeze-drying. The lyophilized powder was redissolved in 0.5 mL of deuterium oxide and freeze-drying was continued. The process was repeated to ensure complete exchange of labile hydrogens. Subsequently, the sample was dissolve in 0.5 mL of deuterium oxide, and 1H NMR, 13C NMR, DEPT135 one-dimensional and two-dimensional spectral measurements were performed at 25°C using a 600 MHz NMR spectrometer.

3. Experimental Results

The hydrogen spectrum signals were primarily concentrated between 3.0 and 5.5 ppm. The signals for sugar ring protons were between δ3.2-4.0 ppm, with main terminal group protons peaks at δ4.42, 4.45, 4.49, 4.50, 4.66, and 4.71, primarily distributed in the 4.3-5.5 ppm region as shown in Figure 16.

Carbon spectral analysis in $^{13}C$ NMR (201 MHz, $D_2O$): The NMR carbon spectrum signals were mainly concentrated between 60-120 ppm. Observations of the carbon spectrum indicated main anomeric carbon signal peaks at δ103.82, 103.89, 104.02, 104.02, 104.12, and 104.21, primarily between δ93-105. Other notable signal peaks were at δ72.72, 74.55, 69.23, 76.08, 60.7, 76.69, 74.09, 77.24, 75.40, 68.82, 74.16, 71.57, 80.15, 76.68, 61.66, 74.76, 84.64,

69.63, 77.03, 62.04, 74.54, 83.90, 71.04, 76.10, 70.28, 74.52, 76.74, 76.41, 70.89, and 68.79 ppm. Based on monosaccharide composition results, the polysaccharide is composed of glucose, indicating the polysaccharide is primarily glucan. This is depicted in Figure 17.

Dept135 spectral analysis showed inverted peaks at 60.70, 68.82, 61.66, 62.04, 70.28, and 68.79 ppm, indicative of chemical shifts for C6, as shown in Figure 18.

Figures 19-22 present HSQC spectra where the anomeric carbon signal at δ103.94 corresponds to anomeric hydrogen signal at δ4.71. HH-COSY identifies H1-2 signal at 4.71/3.44; H2-3 signal at 3.44/3.66; H3-4 signal at 3.66/3.40. We deduce H1, H2, H3, and H4 as δ4.71, 3.44, 3.66, and 3.40 respectively, corresponding to δ104.04, 74.55, 85.9, and 76.63. The corresponding C5 is at 77.41; the chemical shift of C6 is at δ62.04. Therefore, this signal is attributed to the glycosidic bond →3)-β-Glcp-(1→.

Further HSQC observations show anomeric carbon signal at δ104.21, corresponding to anomeric hydrogen signal at δ4.42. HH-COSY identifies H1-2 signal at 4.42/3.23; H2-3 signal at 3.23/3.39; H3-4 signal at 3.39/3.55. We deduce H1, H2, H3, and H4 as δ4.42, 3.23, 3.39, 3.55 respectively, corresponding to C1-4 at δ104.21, 74.52, 76.74, and 76.41. NOESY spectra show correlated peaks at δ4.43 with 3.39, 3.55, 3.75, and 4.11. Dept135 combined with HSQC allows the attribution of δ3.75, 4.11 as peaks for H6a,b; H5 at 3.31 ppm. Corresponding C5 is at δ70.89; C6 chemical shift is at δ70.19, with H6a at δ3.75, 4.11. Therefore, this signal is attributed to the glycosidic bond →6)-β-Glcp-(1→.

Using similar patterns and combining HMBC and NOESY, all glycosidic bond signals are assigned as shown in the following table:

Hydrogen and Carbon Signal Attribution

| Glycosyl residues | H1/C1 | H2/C2 | H3/C3 | H4/C4 | H5/C5 | H6a/C6 | H6b |
|---|---|---|---|---|---|---|---|
| β-D-Glcp-(1→ | 4.66 | 3.52 | 3.43 | 3.47 | 3.57 | 3.81 | 3.64 |
| | 103.89 | 71.72 | 74.55 | 69.23 | 76.08 | 62.01 | |
| →3)-β-D-Glcp-(1→ | 4.71 | 3.44 | 3.66 | 3.4 | 3.39 | 3.62 | 3.81 |
| | 104.02 | 74.55 | 85.9 | 69.63 | 77.41 | 62.04 | |
| →6)-β-D-Glcp-(1→ | 4.42 | 3.23 | 3.39 | 3.55 | 3.31 | 3.75 | 4.12 |
| | 104.21 | 74.52 | 76.74 | 76.41 | 70.89 | 70.19 | |
| →3,6)-β-D-Glcp-(1→ | 4.45 | 3.29 | 3.71 | 3.42 | 3.66 | 3.77 | 4.14 |
| | 104.12 | 74.54 | 85.2 | 71.04 | 77.26 | 70.28 | |
| →4,6)-β-D-Glcp-(1→ | 4.44 | 3.26 | 3.43 | 3.57 | 3.56 | 3.74 | 4.1 |
| | 103.82 | 74.09 | 76.69 | 82.4 | 75.4 | 70.34 | |
| →4)-β-D-Glcp-(1→ | 4.40 | 3.25 | 3.41 | 3.57 | 3.53 | 3.63 | 3.80 |
| | 104.02 | 74.47 | 77.1 | 82.7 | 76.33 | 61.66 | |

**Main Chain Analysis:**

From the HMBC spectra, based on the one-dimensional and two-dimensional NMR spectra, we have attributed the signals of the glycosidic bonds in the polysaccharide: The anomeric hydrogen of the glycosidic bond →6)-β-D-Glcp-(1→ shows correlation signal peaks with its own C6, indicating the presence of a →6)-β-D-Glcp-(1→6)-β-D-Glcp-(1→ linkage.

The anomeric hydrogen of the glycosidic bond →6)-β-D-Glcp-(1→ shows correlation signal peaks with the C6 of →3,6)-β-D-Glcp-(1→, indicating the presence of a →6)-β-D-Glcp-(1→3,6)-β-D-Glcp-(1→ linkage.

The anomeric hydrogen of the glycosidic bond →3,6)-β-D-Glcp-(1→ shows correlation signal peaks with the C6 of →4,6)-β-D-Glcp-(1→, indicating the presence of a →3,6)-β-D-Glcp-(1→4,6)-β-D-Glcp-(1→ linkage.

**Branch Chain Analysis:**

The anomeric carbon of the glycosidic bond →3)-β-D-Glcp-(1→ shows correlation peaks with the H3 of →3,6)-β-D-Glcp-(1→, indicating the presence of a →3)-β-D-Glcp-(1→3,6)-β-D-Glcp-(1→ linkage.

In the NOESY spectra,
The anomeric hydrogen of the glycosidic bond β-D-Glcp-(1→ shows correlation peaks with the H4 of →4)-β-D-Glcp-(1→, indicating the presence of a β-D-Glcp-(1→4)-β-D-Glcp-(1→ linkage.

The anomeric hydrogen of the glycosidic bond β-D-Glcp-(1→ shows correlation peaks with the H3 of →3)-β-D-Glcp-(1→, indicating the presence of a β-D-Glcp-(1→3)-β-D-Glcp-(1→ linkage.

The anomeric hydrogen of the glycosidic bond →4)-β-D-Glcp-(1→ shows correlation peaks with the H4 of →4,6)-β-D-Glcp-(1→, indicating the presence of a →4)-β-D-Glcp-(1→4,6)-β-D-Glcp-(1→ linkage.

Based on the above analysis, we can deduce that the main chain of this polysaccharide is a β-1,6 glucan. Additionally, the β-D-Glcp-(1→3)-β-D-Glcp-(1→ and β-D-Glcp-(1→4)-β-D-Glcp-(1→ are linked to the main chain through the O-3 bond of →3,6)-β-D-Glcp-(1→ and the O-4 bond of →4,6)-β-D-Glcp-(1→, respectively. The condensed molecular structure is illustrated below.

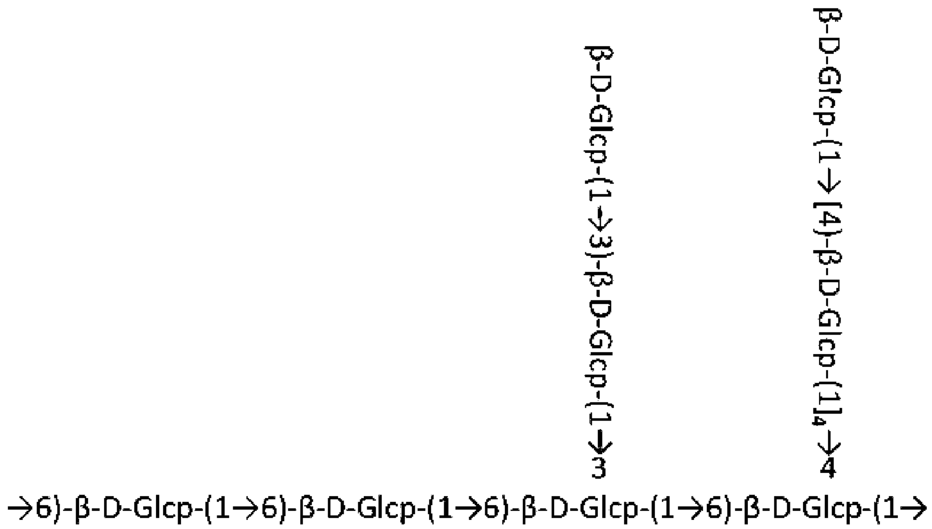

This invention also provides the application of the Ganoderma lucidum polysaccharide GLP-3. The Ganoderma lucidum polysaccharide GLP-3 is characterized by its water solubility, is readily absorbed by the human body, offers antitumor effects, and has proven efficacy in the prevention of tumor development in humans. Notably, when used in combination with chemotherapy drugs, it can alleviate toxic side effects caused by the chemotherapy drugs on the human body, control and reduce tumor masses, and reduce and eliminate cancer cells.

The Ganoderma lucidum polysaccharide GLP-3 is used in medications to inhibit tumor metastasis or to enhance human immune function.

**Experimental Study on the Antitumor Effect of Ganoderma Lucidum Polysaccharide GLP-3 Combined with Chemotherapy on LLC-Bearing Mice and Study Data**

**Experimental Objective**

To study the antitumor effect of Ganoderma lucidum polysaccharide GLP-3 on lung cancer-bearing mice which are prepared by subcutaneously implanting LLC tumor homogenate in the right axilla of C57 mice. This study aims to provide experimental evidence for clinical studies of GLP-3.

**Experimental Materials**

**Test Sample**

Ganoderma lucidum polysaccharide GLP-3, provided by Shenzhen Aolimei Oncology Medical Technology Co., Ltd.

**Positive Control**

Cisplatin, batch number: E2128081, product of Shanghai Aladdin Biochemical Technology Co., Ltd.

**Laboratory Animals**

75 SPF male C57 mice, weighing 14-16 g, supplied by Guangdong Medical Laboratory Animal Center.

Laboratory animal production license number: SCXK (Yue) 2022-0002; laboratory animal quality certification number: 44007200103827.

**Main Reagents**

PBS buffer, prepared by Shenzhen Aolimei Oncology Medical Technology Co., Ltd.; fetal bovine serum, product of Zhejiang Tianhang Biotechnology Co., Ltd.; DMEM culture medium, product of Gibco; 0.25% trypsin, product of Gibco.

**Main Instruments**

Vernier caliper, product of Shanghai Tool Works Co., Ltd.; I-2000 scale, Dongguan Nancheng Changxie Electronic Products Factory; ophthalmic scissors and tweezers, products of Shanghai Jinzhong Medical Instrument Co., Ltd.; CCL-170B-8 $CO_2$ incubator, product of ESCO, Singapore; Luna-II cell counter, product of Nanjing Hengqiao Instrument Co., Ltd.; JRA-35S handheld homogenizer, product of Wuxi Jieruian Instrument Equipment Co., Ltd.

**Experimental Methods**

Healthy LLC cells were inoculated subcutaneously into the left shoulder of 5 healthy male C57 mice. Once tumors reached a volume of 2,000-3,000 $mm^3$, they were harvested and homogenized to prepare a homogenate suspension. This suspension was then injected subcutaneously into the axilla of 60 healthy male C57 mice to establish a solid tumor model. Once all mouse tumors averaged a volume of about 150 $mm^3$, mice were randomized into groups based on tumor volume and were administered the respective drugs or drug solvents via oral gavage or intraperitoneal injection for 19 consecutive days. Longest and shortest diameters of tumors were measured every three days to calculate tumor volume, and mouse weights were recorded every three days. At the end of the experiment, tumors, spleens, and thymuses were harvested and weighed to calculate tumor, spleen, and thymus indices.

**Dosage Design**

Based on previous experimental results, Ganoderma lucidum polysaccharide GLP-3 was administered at a low dose of 50 mg/kg and a high dose of 150 mg/kg. The doses for each respective drug administered in this experiment are shown in Table 1.

Rationale for cisplatin dosage design: Based on the clinical dosage of cisplatin, which should not exceed 100 $mg/m^2$ per person per day, and considering the tolerance of mice to cisplatin, a dose of 4 mg/kg has been selected as the administration dosage.

**Table 1: Experimental groups and dosage design**

| Group | Dose (mg/kg) | Method of administration | Administration volume (mL/10 g) | Frequency of administration |
|---|---|---|---|---|
| Model control group | - | Oral gavage | 0.2 | Once daily |
| Cisplatin | 4 | Intraperitoneal injection | 0.2 | Once every 3 days |
| Cisplatin + GLP-3 low-dose group | 4+50 | Intraperitoneal injection + oral gavage | 0.2+0.2 | Cisplatin, once every 3 days; GLP-3, once daily |
| Cisplatin + GLP-3 high-dose group | 4+150 | Intraperitoneal injection + oral gavage | 0.2+02 | Cisplatin, once every 3 days; GLP-3, once daily |
| Normal group | - | Oral gavage | 0.2 | Once daily |

**Test Indicators**

**Efficacy Indicators**

**Relative tumor growth inhibition rate**

Relative tumor growth inhibition rate (%) = $(1 - T_{RTV} / C_{RTV}) \times 100\%$. Where $T_{RTV}$ is the relative tumor volume in the experimental group, and $C_{RTV}$ is the relative tumor volume in the model control group. Relative tumor volume (RTV) = $V_t / V_0$, where $V_t$ is the tumor volume on day t of dosing, and $V_0$ is the tumor volume at the time of grouping. Evaluation

criteria: A relative tumor growth inhibition rate of ≥ 40% and a statistical analysis with P < 0.05 indicate effective inhibition.

**Tumor growth inhibition rate**

Tumor growth inhibition rate (%) = (1 - T / C) × 100%. Where T represents the average tumor weight in the treatment group, and C represents the average tumor weight in the model control group. Evaluation criteria: A tumor growth inhibition rate of ≥ 40% and a statistical analysis with P < 0.05 indicate effective inhibition.

**Spleen and thymus organ coefficients:** After the last dose, spleen, thymus, and tumor weights are measured, and organ coefficients are calculated.

$$\text{Tumor index (\%)} = (\text{tumor weight / body weight}) \times 100\%.$$

$$\text{Immune organ index (mg/g)} = (\text{organ mass / body weight}) \times 1000.$$

**Data Processing and Statistical Analysis**

Statistical analyses were performed using SPSS 17.0, with the significance level set at P ≤ 0.05. Measurement data were expressed as mean ± standard deviation ($\bar{x} \pm s$). Normality and homogeneity of variance were tested using Leven's test. If data met normality and homogeneity of variance (P > 0.05), one-way ANOVA and LSD test were used for statistical analysis. If data did not meet normality and homogeneity of variance (P < 0.05), the Kruskal-Wallis test was used. If the Kruskal-Wallis test was statistically significant (P < 0.05), comparison analysis was performed using Dunnett's Test (a non-parametric method). Evaluation considered statistical differences and biological significance.

**Experimental Results**

**Animal Mortality**

As shown in Table 2, the mortality rate for all groups of mice in this study was 0.

Table 2: Statistics on the number of surviving animals and mortality rate in each group

| Group | Total number of animals | Number of deaths | Mortality rate (%) | Survival time (days) |
|---|---|---|---|---|
| Model control group | 10 | 0 | 0.0 | 19±0 |
| Cisplatin | 10 | 0 | 0.0 | 19±0 |
| Cisplatin + GLP-3 low-dose group | 10 | 0 | 0.0 | 19±0 |
| Cisplatin + GLP-3 high-dose group | 10 | 0 | 0.0 | 19±0 |
| Normal group | 10 | 0 | 0.0 | 19±0 |

**Effect of Ganoderma Lucidum Polysaccharide GLP-3 Combined with Chemotherapy on Body Weight of LLC-Bearing Mice**

As indicated in Table 3, compared to the normal group, the body weight of mice in the model control group significantly increased on D0 and from D9 to D19. The body weight of mice in the cisplatin group and the cisplatin + GLP-3 high-dose group significantly decreased on D0 and from D6 to D19, and the body weight of mice in the cisplatin + GLP-3 low-dose group significantly decreased on D0 and from D6 to D19.

Compared to the model control group, the body weight of mice in the cisplatin group and the cisplatin + GLP-3 high-dose group significantly decreased from D6 to D19, and the body weight of mice in the cisplatin + GLP-3 low-dose group significantly decreased from D3 to D19.

Compared to the cisplatin group, the body weight of mice in the cisplatin + GLP-3 low-dose group significantly decreased from D12 to D19.

Table 3: Effect of Ganoderma lucidum polysaccharide GLP-3 combined with chemotherapy on body weight of LLC-bearing mice ( $\bar{x} \pm s$)

| Group | Weight (g) | | | | | | |
|---|---|---|---|---|---|---|---|
| | D0 | D3 | D6 | D9 | D12 | D15 | D19 |
| Model control group | **21.6±1.4$^{*}$** | 22.2±1.7 | 22.9±1.6 | **23.9±1.6$^{*}$** | **25.2±1.8$^{*}$** | **26.2±1.8$^{*}$** | **26.6±1.7$^{*}$** |
| Cisplatin | **21.5±1.3$^{*}$** | 20.9±1.3 | **20.4±1.9$^{+*}$** | **18.3±2.6$^{+*}$** | **18.6±2.8$^{+*}$** | **17.7±2.7$^{+*}$** | **16.8±2.8$^{+*}$** |
| Cisplatin + GLP-3 low-dose group | 21.111.4 | **20±1.1$^{+}$** | **19.8±1.5$^{+*}$** | **18.2±1.7$^{+*}$** | **16.7±1.7$^{+\#*}$** | **15.5±1.5$^{+\#*}$** | **14.3±1.8$^{+\#*}$** |
| Cisplatin + GLP-3 high-dose group | **22.4±1$^{*}$** | 22±1.1 | **20.2±1.6$^{+*}$** | **19.7±1.4$^{+*}$** | **19.1±1.3$^{+*}$** | **17.5±1.4$^{+*}$** | **16.4±1.5$^{+*}$** |
| Normal group | **20.2±3.3$^{+\#}$** | 2112.8 | 22±2 | **22.4±2$^{+}$** | **22.6±2.3$^{+}$** | **23.2±2.5$^{+}$** | **23.2±2.7$^{+}$** |

Note: Compared to the model control group, $^{+}P < 0.05$; Compared to the cisplatin group, $^{\#}P < 0.05$; Compared to the normal group, $^{*}P < 0.05$.

**Effect of Ganoderma Lucidum Polysaccharide GLP-3 Combined with Chemotherapy on Tumor Volume in LLC-Bearing Mice**

As shown in Table 4, compared to the model control group, the tumor volume in the cisplatin group was significantly reduced from D9 to D19, and the tumor volume in the cisplatin + GLP-3 low-dose group and the cisplatin + GLP-3 high-dose group was significantly reduced from D6 to D19.

Compared to the cisplatin group, the tumor volume in the cisplatin + GLP-3 low-dose group was significantly reduced from D9 to D19, and the tumor volume in the cisplatin + GLP-3 high-dose group was significantly reduced from D12 to D19.

Table 4: Effect of Ganoderma lucidum polysaccharide GLP-3 combined with chemotherapy on tumor volume in LLC-bearing mice ( x ± s)

| Group | Tumor volume ($mm^3$) | | | | | | |
|---|---|---|---|---|---|---|---|
| | D0 | D3 | D6 | D9 | D12 | D15 | D19 |
| Model control group | 128.8±44. 7 | 304.2±119 .8 | 688.9±265 .9 | 1614±608. 3 | 3036.2±11 77.4 | 5487±135 9 | 7604.1115 14 |
| Cisplatin | 137.9±50 | 300.1±102 .7 | 486.6±188 .9 | **771.3±256 +** | **1173.9±48 8.8$^{+}$** | **1409.5±82 0.5$^{+}$** | **1771.9+10 91.6$^{+}$** |
| Cisplatin + GLP-3 low-dose group | 131±45.8 | 242.2±114 .8 | **354.2±197** + | **488.5±203 .1$^{+\#}$** | **605.2+280 .2$^{+\#}$** | **726.3±353 .2$^{+\#}$** | **635.6±342 .6$^{+\#}$** |
| Cisplatin + GLP-3 high-dose group | 139.7±51. 8 | 252.8±158 | **443.2±296 .4$^{+}$** | **627.5±492 .3$^{+}$** | **837.3±443 .7$^{+\#}$** | **1096.3±63 8.9$^{+\#}$** | **1111.6±81 7$^{+\#}$** |

Note: Compared to the model control group, $^{+}P < 0.05$; Compared to the cisplatin group, $^{\#}P < 0.05$.

**Effect of Ganoderma Lucidum Polysaccharide GLP-3 Combined with Chemotherapy on Relative Tumor Growth Inhibition Rate in LLC-Bearing Mice**

As indicated in Tables 5 and 6, compared to the model control group, the relative tumor growth inhibition rate in the cisplatin group from D9 to D19 was over 40%, specifically 54.6%, 63.8%, 77.3%, and 79.5%; the relative tumor growth inhibition rate in the cisplatin + GLP-3 low-dose group from D6 to D19 was over 50%, specifically 51.9%, 70.0%, 80.1%, 87.4%, and 92.2%; the relative tumor growth inhibition rate in the cisplatin + GLP-3 high-dose group from D6 to D19 was over 40%, specifically 43.3%, 67.1%, 74.9%, 82.3%, and 88.0%.

Compared to the cisplatin group, the relative tumor growth inhibition rate in the cisplatin + GLP-3 low-dose group from D12 to D19 was 44.9%, 44.6%, and 62.3% (P < 0.05), and the relative tumor growth inhibition rate in the cisplatin + GLP-3 high-dose group from D12 to D19 was 30.6%, 22.3%, and 41.6% (P < 0.05).

Table 5: Effect of Ganoderma lucidum polysaccharide GLP-3 combined with chemotherapy on relative tumor growth inhibition rate in LLC-bearing mice (vs. the model control group)

| Group | Relative tumor growth inhibition rate (compared to model control group) (%) | | | | | |
|---|---|---|---|---|---|---|
| | D3 | D6 | D9 | D12 | D15 | D19 |
| Model control group | - | - | - | - | - | - |
| Cisplatin | 4.3 | 30.0 | **54.6**$^{+}$ | **63.8**$^{+}$ | **77.3**$^{+}$ | **79.5**$^{+}$ |
| Cisplatin + GLP-3 low-dose group | 23.1 | **51.9**$^{+}$ | **70.7**$^{+}$ | **80.1**$^{+}$ | **87.4**$^{+}$ | **92.2**$^{+}$ |
| Cisplatin + GLP-3 high-dose group | 22.9 | **43.3**$^{+}$ | **67.1**$^{+}$ | **74.9**$^{+}$ | **82.3**$^{+}$ | **88.0**$^{+}$ |

Note: Compared to the model control group, $^{+}P < 0.05$.

Table 6: Effect of Ganoderma lucidum polysaccharide GLP-3 combined with chemotherapy on relative tumor growth inhibition rate in LLC-bearing mice (vs. the cisplatin group)

| Group | Relative tumor growth inhibition rate (compared to cisplatin group) (%) | | | | | |
|---|---|---|---|---|---|---|
| | D3 | D6 | D9 | D12 | D15 | D19 |
| Cisplatin | - | - | - | - | - | - |
| Cisplatin + GLP-3 low-dose group | 19.6 | 31.4 | 35.5 | **44.9**$^{\#}$ | **44.6**$^{\#}$ | **62.3**$^{\#}$ |
| Cisplatin + GLP-3 high-dose group | 19.5 | 19.0 | 27.5 | **30.6**$^{\#}$ | **22.3**$^{\#}$ | **41.6**$^{\#}$ |

Note: Compared to the cisplatin group, $^{\#}P < 0.05$.

**Effect of Ganoderma Lucidum Polysaccharide GLP-3 Combined with Chemotherapy on Organ Indices and Tumor Growth Inhibition Rates in LLC-Bearing Mice**

As indicated in Table 7, compared to the model group, the tumor index, spleen index, and thymus index were significantly reduced in the cisplatin group, the cisplatin + GLP-3 low-dose group, and the cisplatin + GLP-3 high-dose group. Compared to the cisplatin group, the tumor index was significantly lower in the cisplatin + GLP-3 low-dose group. Compared to the normal group, the spleen index was significantly higher in the model control group, but significantly reduced in the cisplatin + GLP-3 low-dose group; the thymus index was significantly reduced in the model control group, cisplatin group, cisplatin + GLP-3 low-dose group, and cisplatin + GLP-3 high-dose group.

Compared to the model control group, the tumor growth inhibition rate was 74.4% in the cisplatin group, and 91.7% and 84.0% in the cisplatin + GLP-3 low-dose and high-dose groups, respectively. Compared to the cisplatin group, the tumor growth inhibition rate was 67.4% and 37.4% in the cisplatin + GLP-3 low-dose and high-dose groups, respectively.

Table 7: Effect of Ganoderma lucidum polysaccharide GLP-3 combined with chemotherapy on organ indices and tumor growth inhibition rates in LLC-bearing mice

| Group | Tumor index (%) | Spleen index (mg/g) | Thymns index (mg/g) | Tumor growth inhibition rate (%)vs. the model control group | Tumor growth inhibition rate (%)vs. the cisplatin group |
|---|---|---|---|---|---|
| Model control group | 27.11±4.66 | **10.87±1.66**$^{+}$ | **0.86**±**0.37*** | - | - |

(continued)

| Group | Tumor index (%) | Spleen index (mg/g) | Thymns index (mg/g) | Tumor growth inhibition rate (%)vs. the model control group | Tumor growth inhibition rate (%)vs. the cisplatin group |
|---|---|---|---|---|---|
| Cisplatin | **9.09±4.19$^{+}$** | **2.84±0.7$^{+}$** | **0.31**±0.12$^{+*}$ | 74.4 | - |
| Cisplatin + GLP-3 low-dose group | **4.18±2.02$^{+\#}$** | **1.54±0.53$^{++}$** | **0.34±0.17$^{+*}$** | 91.7 | 674 |
| Cisplatin + GLP-3 high-dose group | **6.83±4.24$^{+}$** | **2.49±0.5$^{+}$** | **0.41±0.12$^{+*}$** | 84.0 | 374 |
| Normal group | - | **2.93±0.53$^{+}$** | **1.51±0.35$^{+}$** | - | - |
| Note: Compared to the model control group, $^{+}$P < 0.05; Compared to the cisplatin group, $^{\#}$P < 0.05; Compared to the normal group, $^{*}$P < 0.05. | | | | | |

Figures 23, 24, 25, and 26 show images of the tumor-bearing mice, corresponding to the groups as follows: Figure 23: model control group, Figure 24: cisplatin group, Figure 25: cisplatin + GLP-3 low-dose group, Figure 26: cisplatin + GLP-3 high-dose group.

Figures 27, 28, 29, and 30 show images of the tumors in the tumor-bearing mice, corresponding to the groups as follows: Figure 27: model control group, Figure 28: cisplatin group, Figure 29: cisplatin + GLP-3 low-dose group, Figure 30: cisplatin + GLP-3 high-dose group.

Conclusion

Ganoderma Lucidum Polysaccharide GLP-3 combined with cisplatin significantly inhibits tumor growth in LLC-bearing mice and exhibits a significant synergistic effect.

**Experimental Study on the Antitumor Effect of Ganoderma Lucidum Polysaccharide GLP-3 Combined with Chemotherapy on H22-Bearing Mice and Study Data**

**Experimental Objective**

To study the effect of Ganoderma lucidum polysaccharide GLP-3 on hepatoma-bearing mice which are prepared by implanting H22 hepatoma tumor homogenate in the right axilla of C57 mice. This study aims to provide experimental evidence for clinical studies of GLP-3.

**Experimental Materials**

**Test Sample**

Ganoderma lucidum polysaccharide GLP-3, provided by Shenzhen Aolimei Oncology Medical Technology Co., Ltd.

**Positive Control**

Cisplatin, batch number: E2128081, product of Shanghai Aladdin Biochemical Technology Co., Ltd.

**Laboratory Animals**

65 SPF male C57 mice, weighing 16-18 g, supplied by Guangdong Medical Laboratory Animal Center. Laboratory animal production license number: SCXK (Yue) 2022-0002; laboratory animal quality certification number: 44007200103599.

**Main Reagents**

PBS buffer, prepared by Shenzhen Aolimei Oncology Medical Technology Co., Ltd.; fetal bovine serum, product of Zhejiang Tianhang Biotechnology Co., Ltd.; 1640 culture medium, product of Gibco; 0.25% trypsin, product of Gibco.

**Main Instruments**

Vernier caliper, product of Shanghai Tool Works Co., Ltd.; I-2000 scale, Dongguan Nancheng Changxie Electronic Products Factory; ophthalmic scissors and tweezers, products of Shanghai Jinzhong Medical Instrument Co., Ltd.; CCL-170B-8 $CO_2$ incubator, product of ESCO, Singapore; Luna-II cell counter, product of Nanjing Hengqiao Instrument Co., Ltd.; JRA-35S handheld homogenizer, product of Wuxi Jieruian Instrument Equipment Co., Ltd.

**Experimental Methods**

A homogenate suspension of H22 hepatoma solid tumors was injected subcutaneously under the axilla of 50 healthy male C57 mice to create a solid tumor model. Once all mouse tumors averaged a volume of about 200 $mm^3$, mice were randomized into groups based on tumor volume and were administered the respective drugs or drug solvents via oral gavage or intraperitoneal injection for 22 consecutive days. Longest and shortest diameters of tumors were measured every three days to calculate tumor volume, and mouse weights were recorded every three days. At the end of the experiment, tumors, spleens, and thymuses were harvested and weighed to calculate tumor, spleen, and thymus indices.

**Dosage Design**

Based on previous experimental results, Ganoderma lucidum polysaccharide GLP-3 was administered at a low dose of 50 mg/kg and a high dose of 150 mg/kg as shown in Table 8.

Rationale for cisplatin dosage design: Based on the clinical dosage of cisplatin, which should not exceed 100 $mg/m^2$ per person per day, and considering the tolerance of mice to cisplatin, a dose of 3 mg/kg has been selected as the administration dosage.

Table 8: Experimental groups and dosage design

| Group | Dose (mg/kg) | Method of administration | Administration volume (mL/10 g) | Frequency of administration |
|---|---|---|---|---|
| Model control group | - | Oral gavage | 0.2 | Once daily |
| Cisplatin | 3 | Intraperitoneal injection | 0.2 | Once every 3 days |
| Cisplatin + GLP-3 low-dose group | 3+50 | Intrapertoneal injection + oral gavage | 0.2+0.2 | Cisplatin, once every 3 days; GLP-3, once daily |
| Cisplatin + GLP-3 high-dose group | 3+150 | Intraperitoneal injection + oral gavage | 0.2+0.2 | Cisplatin, once every 3 days; GLP-3, once daily |
| Normal group | - | Oral gavage | 0.2 | Once daily |

**Test Indicators**

**Efficacy Indicators**

**Relative tumor growth inhibition rate**

Relative tumor growth inhibition rate (%) = (1 - $T_{RTV}$ / $C_{RTV}$) × 100%. Where $T_{RTV}$ is the relative tumor volume in the experimental group, and $C_{RTV}$ is the relative tumor volume in the model control group. Relative tumor volume (RTV) = $V_t$ / $V_0$, where $V_t$ is the tumor volume on day t of dosing, and $V_0$ is the tumor volume at the time of grouping. Evaluation criteria: A relative tumor growth inhibition rate of ≥ 40% and a statistical analysis with $P < 0.05$ indicate effective inhibition.

**Tumor growth inhibition rate**

Tumor growth inhibition rate (%) = (1 - T / C) × 100%. Where T represents the average tumor weight in the treatment group, and C represents the average tumor weight in the model control group. Evaluation criteria: A tumor

growth inhibition rate of ≥ 40% and a statistical analysis with P < 0.05 indicate effective inhibition.

**Spleen and thymus organ coefficients:** After the last dose, spleen, thymus, and tumor weights are measured, and organ coefficients are calculated.

$$\text{Tumor index (\%)} = (\text{tumor weight} / \text{body weight}) \times 100\%.$$

$$\text{Immune organ index (mg/g)} = (\text{organ mass} / \text{body weight}) \times 1000.$$

**Data Processing and Statistical Analysis**

Statistical analyses were performed using SPSS 17.0, with the significance level set at P ≤ 0.05. Measurement data were expressed as mean ± standard deviation ($\bar{x} \pm s$). Normality and homogeneity of variance were tested using Leven's test. If data met normality and homogeneity of variance (P > 0.05), one-way ANOVA and LSD test were used for statistical analysis. If data did not meet normality and homogeneity of variance (P < 0.05), the Kruskal-Wallis test was used. If the Kruskal-Wallis test was statistically significant (P < 0.05), comparison analysis was performed using Dunnett's Test (a non-parametric method). Evaluation considered statistical differences and biological significance.

**Experimental Results**

**Animal Mortality**

As shown in Table 9, the mortality rate for the model control group of mice was 50%, while the mortality rates for all other groups were 0.

Table 9: Statistics on the number of surviving animals and mortality rate in each group

| Group | Total number of animals | Number of deaths | Mortality rate (%) | Survival time (days) |
|---|---|---|---|---|
| Model control group | 10 | 5 | 50.0 | 18.114.4 |
| Cisplatin | 10 | 0 | 0.0 | 22±0 |
| Cisplatin + GLP-3 low-dose group | 10 | 0 | 0.0 | 22±0 |
| Cisplatin + GLP-3 high-dose group | 10 | 0 | 0.0 | 22±0 |
| Normal group | 10 | 0 | 0.0 | 22±0 |

**Effect of Ganoderma Lucidum Polysaccharide GLP-3 Combined with Chemotherapy on Body Weight of H22-Bearing Mice**

As indicated in Table 10, compared to the model control group, the body weight of mice in the cisplatin group significantly decreased on D6 and from D12 to D22. The body weight of mice in the cisplatin + GLP-3 low-dose group significantly decreased from D6 to D22, and the body weight of mice in the cisplatin + GLP-3 high-dose group significantly decreased from D3 to D22.

Compared to the cisplatin group, the body weight of mice in the cisplatin + GLP-3 low-dose group significantly decreased on D3 and D6 and from D12 to D22, and the body weight of mice in the cisplatin + GLP-3 high-dose group significantly decreased from D0 to D22.

Table 10: Effect of Ganoderma lucidum polysaccharide GLP-3 combined with chemotherapy on body weight of H22-bearing mice ( x ± s)

| Group | Weight (g) | | | | | | | |
|---|---|---|---|---|---|---|---|---|
| | D0 | D3 | D6 | D9 | D12 | D15 | D18 | D22 |
| Model control group | 17.9±1.4 | 19.2±1.4 | 19.9=1.5 | 20.8+1.6 | 22.6±1.8 | 23.4±1.8 | 24.4±3.1 | 27.2±3.2 |
| Cisplatin | **18.9±0.8**$^{+}$ | 19.9±0.7 | **19.5**±0.**6**$^{+}$ | 19.6±0.8 | **19.5±0.8**$^{+}$ | **19.2±0.7**$^{+}$ | **18.5±0.9**$^{+}$ | **19.7±1.4**$^{+}$ |
| Cisplatin + GLP-3 low-dose group | 18.4±1.1 | **18.7**±0.**9**$^{\#}$ | **18.4**±0.**6**$^{+\#}$ | **18.4±0.6**$^{+}$ | **18**±0.6$^{+\#}$ | **16.8±0.9**$^{+\#}$ | **16.6±0.7**$^{+\#}$ | **17.3±0.9**$^{+\#}$ |
| Cisplatin + GLP-3 high-dose group | **17.7±1**$^{\#}$ | **17.8±1**$^{+\#}$ | **17.5±1.1**$^{+\#}$ | **17.5±1.4**$^{+\#}$ | **16.5±1.4**$^{+\#}$ | **15.3±1.5**$^{+\#}$ | **15±1.8**$^{+\#}$ | 15.7±2.2$^{+\#}$ |
| Normal group | 17.2±2.3 | 18.4±1.9 | 19.4±1.8 | 20.1±1.5 | 20.6±1.6 | 20.7±1.7 | 19.1±6.4 | 19.6±6.6 |

Note: Compared to the model control group, $^{+}P < 0.05$; Compared to the cisplatin group, $^{\#}P < 0.05$; Compared to the normal group, $^{*}P < 0.05$.

**Effect of Ganoderma Lucidum Polysaccharide GLP-3 Combined with Chemotherapy on Tumor Volume in H22-Bearing Mice**

As shown in Table 11, compared to the model control group, the tumor volume in the cisplatin group was significantly reduced from D9 to D22, and the tumor volume in the cisplatin + GLP-3 low-dose group and the cisplatin + GLP-3 high-dose group was significantly reduced on D3 and from D9 to D22.

Compared to the cisplatin group, the tumor volume in the cisplatin + GLP-3 low-dose group and high-dose group was significantly reduced from D9 to D22.

Table 11: Effect of Ganoderma lucidum polysaccharide GLP-3 combined with chemotherapy on tumor volume in H22-bearing mice ( x ± s)

| Group | Tmnor volume ($mm^3$) | | | | | | | |
|---|---|---|---|---|---|---|---|---|
| | DO | D3 | D6 | D9 | D12 | D15 | D18 | D22 |
| Model control group | 186.3±48.6 | 389.6±120.1 | 425.7±149.3 | 1211.8±366.9 | 2207.8±1041.7 | 3223.5±1629.5 | 4096.7±1889.4 | 6465.9±3028.1 |
| Cisplatin | 188.2±48.7 | 338.6±69.1 | 356.9±77.9 | **598.5±219.2**$^{+}$ | **825.9±310.3**$^{+}$ | **900.4±376.4**$^{+}$ | **1098.3±3967**$^{+}$ | **1526.5±535.7**$^{+}$ |
| Cisplatin + GLP-3 low-dose group | 183.6±62.6 | **257.9±43.7**$^{+}$ | 325.5±99.3 | **315±93.8**$^{+\#}$ | **470.2±161.3**$^{+\#}$ | **447.6±137.9**$^{+\#}$ | **556.9±319.2**$^{+\#}$ | **965±514.7**$^{+\#}$ |
| Cisplatin + GLP-3 high-dose group | 185±48.3 | **265.7±85.9**$^{+}$ | 260.3±128.4 | **292.9±171.9**$^{+\#}$ | **389.5**±285,1$^{+\#}$ | **378.3±1352.2**$^{+\#}$ | **373.1±268.8**$^{+\#}$ | **728.9±646.9**$^{+\#}$ |

Note: Compared to the model control group, $^{+}P < 0.05$; Compared to the cisplatin group, $^{\#}P < 0.05$.

**Effect of Ganoderma Lucidum Polysaccharide GLP-3 Combined with Chemotherapy on Relative Tumor Growth Inhibition Rate in H22-Bearing Mice**

As indicated in Tables 12 and 13, compared to the model control group, the relative tumor growth inhibition rate in the cisplatin group from D9 to D22 was over 50%, specifically 53.1%, 64.4%, 73.1%, 71.6%, and 75.0%; the relative tumor growth inhibition rate in the cisplatin + GLP-3 low-dose group from D9 to D22 was over 70%, specifically 72.1%, 75.9%, 84.2%, 81.7%, and 80.4%; the relative tumor growth inhibition rate in the cisplatin + GLP-3 high-dose group from D9 to D22 was over 75%, specifically 77.6%, 83.4%, 89.2%, 90.4%, and 88.0%.

Compared to the cisplatin group, the relative tumor growth inhibition rate in the cisplatin + GLP-3 low-dose group from D9 to D22 was 40.5%, 32.3%, 41.2%, 35.7%, and 21.4% (P < 0.05), and the relative tumor growth inhibition rate in the cisplatin + GLP-3 high-dose group from D9 to D22 was over 50%, specifically 52.3%, 53.3%, 59.9%, 66.2%, and 51.9% (P < 0.05).

Table 12: Effect of Ganoderma lucidum polysaccharide GLP-3 combined with chemotherapy on relative tumor growth inhibition rate in H22-bearing mice (vs. the model control group)

| Group | Relative tumor growth inhibition rate (compared to model control group) (%) | | | | | | |
|---|---|---|---|---|---|---|---|
| | D3 | D6 | D9 | D12 | D15 | D18 | D22 |
| Model control group | - | - | - | - | - | - | - |
| Cisplatin | 11.7 | 19.5 | **53.1**$^{+}$ | **64.4**$^{+}$ | **73.1**$^{+}$ | **71.6**$^{+}$ | **75.0**$^{+}$ |
| Cisplatin + GLP-3 low-dose group | **28.0**$^{+}$ | 15.1 | **72.1**$^{+}$ | **75.9**$^{+}$ | **84.2**$^{+}$ | **81.7**$^{+}$ | **80.4**$^{+}$ |
| Cisplatin + GLP-3 high-dose group | **32.2**$^{+}$ | 41.3 | **77.6**$^{+}$ | **83.4**$^{+}$ | **89.2**$^{+}$ | **90.4**$^{+}$ | **88.0**$^{+}$ |

Note: Compared to the model control group, $^{+}P < 0.05$.

Table 13: Effect of Ganoderma lucidum polysaccharide GLP-3 combined with chemotherapy on relative tumor growth inhibition rate in H22-bearing mice (vs. the cisplatin group)

| Group | Relative tumor growth inhibition rate (compared to cisplatin group) (%) | | | | | | |
|---|---|---|---|---|---|---|---|
| | D3 | D6 | D9 | D12 | D15 | D18 | D22 |
| Cisplatin | - | - | - | - | - | - | - |
| Cisplatin + GLP-3 low-dose group | 18.5 | -5.5 | **40.5**$^{\#}$ | **32.3**$^{\#}$ | **41.2**$^{\#}$ | **35.7**$^{\#}$ | **21.4**$^{\#}$ |
| Cisplatin + GLP-3 high-dose group | 23.3 | 27.0 | **52.3**$^{\#}$ | **53.3**$^{\#}$ | **59.9**$^{\#}$ | **66.2**$^{\#}$ | **51.9**$^{\#}$ |

Note: Compared to the cisplatin group, $^{\#}P < 0.05$.

**Effect of Ganoderma Lucidum Polysaccharide GLP-3 Combined with Chemotherapy on Organ Indices and Tumor Growth Inhibition Rates in H22-Bearing Mice**

As indicated in Table 14, compared to the model group, the tumor index, spleen index, and thymus index were significantly reduced in the cisplatin group, the cisplatin + GLP-3 low-dose group, and the cisplatin + GLP-3 high-dose group. Compared to the cisplatin group, the tumor index was significantly reduced in the cisplatin + GLP-3 high-dose group, and the spleen index was significantly reduced in the cisplatin + GLP-3 low-dose group and the cisplatin + GLP-3 high-dose group. Compared to the normal group, the spleen index was significantly reduced in the model control group; the thymus index was significantly reduced in the cisplatin group, the cisplatin + GLP-3 low-dose group, and the cisplatin + GLP-3 high-dose group.

Compared to the model control group, the tumor growth inhibition rate was 75.7% in the cisplatin group, and 82.8% and 85.0% in the cisplatin + GLP-3 low-dose and high-dose groups, respectively. Compared to the cisplatin group, the tumor growth inhibition rate was 29.4% and 38.4% in the cisplatin + GLP-3 low-dose and high-dose groups, respectively.

Table 14: Effect of Ganoderma lucidum polysaccharide GLP-3 combined with chemotherapy on organ indices and tumor growth inhibition rates in H22-bearing mice

| Group | Tmnor index (%) | Spleen index (mg/g) | Thymus index (mg/g) | Tumor growth inhibition rate (%) vs. the model control group | Tumor growth inhibition rate (%) vs. the cisplatin group |
|---|---|---|---|---|---|
| Model control group | 19.8±9.5 | **6.3±2.5***$^{*}$ | 1.1±0.4 | - | - |
| Cisplatin | **6.6±2.2**$^{+}$ | **3.4±0.3**$^{+}$ | **0.6±0.1**$^{*}$ | 75.7 | - |
| Cisplatin + GLP-3 low-dose group | **5.2±2.5**$^{+}$ | **3±0.4**$^{+\#}$ | **0.8±0.2**$^{*}$ | 82.8 | 29.4 |
| Cisplatin + GLP-3 high-dose group | **4.7±4.1**$^{+\#}$ | **2.6±0.4**$^{+\#}$ | **0.7±0.4**$^{*}$ | 85.0 | 38.4 |
| Normal group | - | **2.5±0.7**$^{+}$ | 1.6±0.5 | - | - |

Note: Compared to the model control group, $^{+}P < 0.05$; Compared to the cisplatin group, $^{\#}P < 0.05$; Compared to the normal group, $^{*}P < 0.05$.

Figures 31-34 show images of the tumor-bearing mice, corresponding to the groups as follows: Figure 31: model control group, Figure 32: cisplatin group, Figure 33: cisplatin + GLP-3 low-dose group, Figure 34: cisplatin + GLP-3 high-dose group.

Figures 35-38 show images of the tumors in the tumor-bearing mice, corresponding to the groups as follows: Figure 35: model control group, Figure 36: cisplatin group, Figure 37: cisplatin + GLP-3 low-dose group, Figure 38: cisplatin + GLP-3 high-dose group.

Conclusion

Ganoderma Lucidum Polysaccharide GLP-3 combined with cisplatin significantly inhibits tumor growth in H22-bearing mice and exhibits a significant synergistic effect.

**Experimental Study on the Antitumor Effect of Ganoderma Lucidum Polysaccharide GLP-3 Combined with Radiotherapy on Orthotopic H22-Bearing Mice and Study Data**

**Experimental Objective**

To study the effect of Ganoderma lucidum polysaccharide GLP-3 combined with chemotherapy on orthotopic H22-bearing mice which are prepared by implanting H22 hepatoma tumors in the right axilla of C57 mice. This study aims to provide experimental evidence for clinical studies of GLP-3.

**Experimental Materials**

**Test Sample**

Ganoderma lucidum polysaccharide GLP-3, batch number: ALM20200518A1, provided by Shenzhen Aolimei Oncology Medical Technology Co., Ltd.

**Positive Control**

Kanglaite soft capsules, batch number: 20211009, Zhejiang Kanglaite Pharmaceutical Co., Ltd.; cisplatin injection, batch number: 601211204, Jiangsu Hansoh Pharmaceutical Co., Ltd.

**Laboratory Animals**

100 SPF-grade male C57 mice, weighing 12-15 g, provided by Hunan SJA Laboratory Animal Co., Ltd. Laboratory animal production license number: SCXK (Xiang) 2019-0004; laboratory animal quality certification numbers: No430727221101898084 and No430727221101898112. The animals were housed in Barrier Environment Laboratory D of Hunan Puruima Pharmaceutical Research Center Co., Ltd., under the laboratory animal use license number: SYXK (Xiang) 2020-0015.

**Main Reagents**

0.9% sodium chloride injection, batch number: 21071401C, Hunan Kangyuan Pharmaceutical Co., Ltd.; diluent for veterinary blood cell analysis, batch number: 2022052603; veterinary ALT assay kit, batch number: 201751; AST assay kit, batch number: 201750; CRE assay kit, batch number: 111644; BUN assay kit, batch number: 201749, all manufactured by Wako Pure Chemical Industries, Ltd. of Japan.

**Main Instruments**

AR223CN Electronic Scale, Ohaus Instruments (Changzhou) Co., Ltd.; LABOSPECT003 Automatic Biochemical Analyzer, Hitachi, Japan; AniView100 Multimodal Animal In Vivo Imaging System, ANDOR; TDZ5-WS Benchtop Multi-Tube Automatic Balance Centrifuge, Hunan Kaida Industrial Development Co., Ltd.; ME2002E Electronic Scale, Shimadzu Corporation, Japan; Flow Cytometer, BD Biosciences; ASP200S Fully Automatic Tissue Dehydrator, ASP300S Fully Automatic Tissue Dehydrator, TP1020 Fully Automatic Dehydrator, HI1210 Slide Spreader, HI1220 Slide Dryer, RM2235 Paraffin Microtome, EG1150H+C Tissue Embedding Station, AutoStainer XL Automatic Slide Stainer + CV5030 Automatic Cover Slipper, BX43 Biological Microscope + MD50 Digital Imaging System, CX31 Biological Microscope, all from Leica, Germany.

**Experimental Methods**

Liver cancer (H22) cells, labeled with Luc fluorescent marker at a concentration of $1\times10^7$ cells/mL, were initially inoculated into the peritoneal cavity of 8 male C57 mice. After the development of ascites, the ascitic fluid was aseptically extracted, washed with HBSS buffer, centrifuged to discard the supernatant, and stained with Trypan Blue, and cells in the ascitic fluid were counted under a microscope. The cell concentration was adjusted to $1\times10^{13}$/mL with HBSS buffer and inoculated into the right hepatic region of 80 male C57 mice, at a volume of 10 $\mu$L per mouse, to prepare the orthotopic tumor-bearing mice. One week later, the liver tumor formation in mice was detected using a small animal in vivo imaging system. Based on tumor size, the mice were randomized into groups: model control group, radiotherapy group (2 Gy/d), radiotherapy + cisplatin group (2 Gy + 4 mg/kg), Kanglaite soft capsule group (1,404 mg/kg), radiotherapy + Kanglaite soft capsule group (2 Gy + 1,404 mg/kg), radiotherapy + GLP-3 low-dose group (2 Gy + 130 mg/kg), and radiotherapy + GLP-3 high-dose group (2 Gy + 1,170 mg/kg), with 10 mice per group. An additional 10 mice served as the normal control group. Except for the normal control group, all other animals underwent radiotherapy using an animal radiotherapy instrument. The animals were anesthetized, placed in homemade lead clothing, and the tumor tissue was exposed for radiation treatment at an intensity of 2 Gy/day for 5 consecutive days. The normal control group and the model control group were administered pure water by oral gavage. The radiotherapy + cisplatin group received intraperitoneal injections of cisplatin, and the other groups were administered their respective drug solutions by oral gavage (20 mL/kg) or intraperitoneal injection (10 mL/kg), once daily for 14 consecutive days. After the last dose, blood was collected from the orbital plexus to test blood WBC, RBC, liver and kidney function indicators (ALT, AST, BUN, CRE), and $CD3^+/CD4^+$, $CD3^+/CD8^+$ ratios. The spleen, thymus, and tumors were weighed to calculate organ coefficients, and the liver, spleen, and thymus underwent histopathological examination.

**Dosage Design**

Based on previous experimental results, Ganoderma lucidum polysaccharide GLP-3 was administered at a low dose of 130 mg/kg and a high dose of 1170 mg/kg as shown in Table 15.

The proposed clinical dose for Kanglaite soft capsules is 0.45 g per capsule, 6 capsules per dose, 4 doses per day, which totals 10.8 g per day. When converted to an equivalent mouse dose based on body surface area, it is calculated as 10.8 g/day $\times$ 0.0026 / 0.02 kg = 1,404 mg/kg. This study used the proposed clinical dose as a basis for the experiment.

The radiotherapy intensity for this study was designed to be 2 Gy per day.

Table 15: Experimental groups and dosage design

| Group | Dose (mg/kg) | Method of administration | Frequency of admistration |
|---|---|---|---|
| Normal control group | - | Oral gavage | Once daily |
| Model control group | - | Oral gavage | Once daily |
| Radiotherapy group | 2Gy | Oral gavage | Radiotherapy: 2 Gy/d, once daily |
| Radiotherapy + cisplatin group | 2Gy+4 | Intraperitoneal injection | Radiotherapy: 2 Cy/d; cisplatin: once every 3 days |
| Kanglaite soft capsule group | 1404 | Oral gavage | Once daily |

(continued)

| Group | Dose (mg/kg) | Method of administration | Frequency of admistration |
|---|---|---|---|
| Radiotherapy + Kanglaite soft capsule group | 2Gy+1404 | Oral gavage | Radiotherapy: 2 Gy/d; Kanglaite soft capsule: once daily |
| Radiotherapy + GLP-3 low-dose group | 2Gy+130 | Oral gavage | Radiotherapy: 2 Gy/d: GLP-3: once daily |
| Radiotherapy + GLP-3 high-dose group | 2Gy+1170 | Oral gavage | Radiotherapy: 2 Gy/d; GLP-3: once daily |

**Test Indicators**

**Efficacy Indicators**

**Animal survival and general condition:** Weight was recorded weekly, along with any deaths among the animals.

**Tumor volume measurement:** Tumor volume changes were measured weekly using small animal in vivo imaging technology.

**Hematological tests:** After the last dose, routine blood tests (WBC and RBC) and biochemical tests (liver and kidney functions) were performed.

**Immune organs:** The thymus, spleen, tumor, and liver were weighed, and organ coefficients were calculated as follows: organ coefficient (%) = organ weight / fasting body weight × 100%.

**$CD4^+$ and $CD8^+$ content measurement:** $^+/CD4^+$ and $CD3^+/CD8^+$ in the blood were measured using flow cytometry.

**Data Processing and Statistical Analysis**

Significant figures of the study data were rounded according to the nearest whole number. Statistical analysis was conducted in accordance with the center's *SOP,* using SPSS software. Measurement data are expressed as mean ± standard deviation ($\bar{x} \pm$ **s**), and normality and homogeneity of variance were tested using Leven's test. If not statistically significant ($P > 0.05$), one-way ANOVA was used for statistical analysis. If ANOVA was statistically significant ($P \leq 0.05$), the LSD test (parametric method) was used for comparison analysis. If the variance was not homogeneous ($P \leq 0.05$), the Kruskal-Wallis test was employed. If the Kruskal-Wallis test was statistically significant ($P \leq 0.05$), the Dunnett's Test (non-parametric method) was used for comparison analysis. Statistical significance was determined at a = 0.05, where $P \leq 0.05$ indicates statistical significance and $P \leq 0.01$ indicates highly significant differences.

**Experimental Results**

**Animal Mortality**

In the model control group, mice 2M07/2M08 and 2M05 died on D4 and D10, respectively. In the radiotherapy group, mice 3M02/3M10, 3M07, 3M09/3M01, and 3M05 died on D3, D4, D5, and D10, respectively. In the radiotherapy + cisplatin group, mice 4M03/4M09, 4M06, 4M10, 4M07, and 4M04 died on D4, D6, D7, D8, and D13, respectively. In the Kanglaite soft capsule group, mice 5M03, 5M07, and 5M08 died on D8, D10, and D14, respectively. In the radiotherapy + Kanglaite soft capsule group, mice 6M10, 6M08/6M02, 6M05, 6M06, and 6M01/6M07 died on D7, D8, D10, D13, and D14, respectively. In the radiotherapy + GLP-3 low-dose group, mouse 7M01 died on D8. In the radiotherapy + GLP-3 high-dose group, mice 8M03, 8M08, 8M06, and 8M02 died on D9, D10, D13, and D14, respectively.

The mortality rates for each group were as follows: 0%, 30%, 60%, 60%, 30%, 70%, 10%, and 50%.

Table 16: Statistics on the number of surviving animals and mortality rate in each group

| Group | Dose (mg/kg) | Number of animals | Number of deaths | Number of surviving animals | Mortalit y rate (%) |
|---|---|---|---|---|---|
| Normal control group | - | 10 | 0 | 10 | 0 |
| Model control group | - | 10 | 3 | 7 | 30 |
| Radiotherapy group | 2Gy | 10 | 6 | 4 | 60 |

(continued)

| Group | Dose (mg/kg) | Number of animals | Number of deaths | Number of surviving animals | Mortalit y rate (%) |
|---|---|---|---|---|---|
| Radiotherapy + cisplatin group | 2Gy+4 | 10 | 6 | 4 | 60 |
| Kanglaite soft capsule group | 1404 | 10 | 3 | 7 | 30 |
| Radiotherapy + Kanglaite soft capsule group | 2Gy+1404 | 10 | 7 | 3 | 70 |
| Radiotherapy + GLP-3 low-dose group | 2Gy+130 | 10 | 1 | 9 | 10 |
| Radiotherapy + GLP-3 high-dose group | 2Gy+1170 | 10 | 4 | 6 | 50 |

**Effect of Ganoderma Lucidum Polysaccharide GLP-3 Combined with Radiotherapy on Body Weight of Orthotopic H22-Bearing Mice**

As shown in Table 17, compared to the normal control group, the body weight of the mice in the model control group significantly decreased after administration on weeks 0, 1, and 2 ($P \leq 0.01$). Compared to the model control group, the body weight of mice in the radiotherapy group, radiotherapy + cisplatin group, Kanglaite soft capsule group, and GLP-3 low-dose group significantly decreased after administration on weeks 1 and 2 ($P \leq 0.05$ or $P \leq 0.01$); the body weight of mice in the radiotherapy + GLP-3 high-dose group significantly decreased in week 1 ($P \leq 0.05$). Compared to the radiotherapy group, the body weight of mice in the Kanglaite soft capsule group significantly increased after administration on weeks 1 and 2 ($P \leq 0.05$ or $P \leq 0.01$). Compared to the radiotherapy + cisplatin group, the body weight of mice in the radiotherapy + GLP-3 low-dose and high-dose groups, and the Kanglaite soft capsule group significantly increased after administration on weeks 1 and 2 ($P \leq 0.05$ or $P \leq 0.01$). No significant differences were observed in the remaining groups.

Table 17: Effect of Ganoderma lucidum polysaccharide GLP-3 combined with radiotherapy on body weight of orthotopic H22-bearing mice (Mean ± SEM)

| Group | Weight (g) | | |
|---|---|---|---|
| | WO | W1 | W2 |
| Normal control group | 20.2 ± 0.3 | 22.0 ± 0.4 | 23.0 ± 0.4 |
| Model control group | **17.0 ± 0.6$^{++}$** | **18.8 ± 0.7$^{++}$** | **19.0 ± 0.4$^{++}$** |
| Radiotherapy group | **17.1 ± 0.5$^{++}$** | **14.9 ± 0.8$^{**}$** | **16.3 ± 1.4$^{**}$** |
| Radiotherapy + cisplatin group | **17.1 ± 0.4$^{++}$** | **13.6 ± 0.9$^{**}$** | **14.6 ± 1.0$^{**}$** |
| Kanglaite soft capsule group | **16.9 ± 0.7$^{++}$** | **18.7 ± 0.5$^{\#\#\&\&}$** | **19.0 ± 0.5$^{\#\#\&\&}$** |
| Radiotherapy + Kanglaite soft capsule group | **17.0 ± 0.6$^{++}$** | **14.9 ± 0.9$^{**}$** | **16.3 ± 0.1$^{*}$** |
| Radiotherapy + GLP-3 low-dose group | **17.0 ± 0.5$^{++}$** | **15.4 ± 0.5$^{**\&}$** | **17.0 ± 0.6$^{*\&}$** |
| Radiotherapy + GLP-3 high-dose group | **16.9 ± 0.6$^{++}$** | **15.7 ± 0.6$^{**\&}$** | **17.3 ± 0.7$^{\&}$** |

Note: Compared to the normal control group, $^{++}P \leq 0.01$; compared to the model control group, $^{*}P \leq 0.05$, $^{**}P \leq 0.01$; compared to the radiotherapy group, $^{\#}P \leq 0.05$, $^{\#\#}P \leq 0.01$; compared to the radiotherapy + cisplatin group, $^{\&}P \leq 0.05$, $^{\&\&}P \leq 0.01$.

**Effect of Ganoderma Lucidum Polysaccharide GLP-3 Combined with Radiotherapy on Tumors in Orthotopic H22-Bearing Mice**

As shown in Figures 39, 40, and Table 18, compared to the normal control group, the tumors in the mice of the model control group increased significantly ($P \leq 0.01$); compared to the model control group, the tumors in mice of the radiotherapy + GLP-3 low-dose and high-dose groups, and the radiotherapy + cisplatin group significantly decreased in week 2 ($P \leq 0.05$ or $P \leq 0.01$). Compared to the Kanglaite soft capsule group, the tumors in the mice of the radiotherapy + GLP-3 high-dose group significantly decreased in week 2 ($P \leq 0.05$). No significant differences were observed in the other groups.

As shown in Figure 39, the corresponding groups are as follows: A: normal group, B: model control group, C: radiotherapy group, D: radiotherapy + cisplatin group, E: Kanglaite soft capsule group, F: radiotherapy + Kanglaite soft capsule group, G: radiotherapy + GLP-3 low-dose group, H: radiotherapy + GLP-3 high-dose group.

As shown in Figure 40, the corresponding groups are as follows: 1: normal group, 2: model control group, 3: radiotherapy group, 4: radiotherapy + cisplatin group, 5: Kanglaite soft capsule group, 6: radiotherapy + Kanglaite soft

capsule group, 7: radiotherapy + GLP-3 low-dose group, 8: radiotherapy + GLP-3 high-dose group.

Table 18: Effect of Ganoderma lucidum polysaccharide GLP-3 combined with radiotherapy on tumors in orthotopic H22-bearing mice (Mean ± SEM)

| Group | WO | | W1 | | W2 | |
|---|---|---|---|---|---|---|
| | Tumor (P/s/cm$^2$/sr) | Niunber of animals | Tumor (P/s/cm$^2$/sr) | Number of animals | Tumor (P/s/cm$^2$/sr) | Number of animals |
| Normal control group | 0 ± 0 | 10 | 0 ± 0 | 10 | 0 ± 0 | 10 |
| Model control group | 764020 ± 667957$^{++}$ | 10 | **12287500 ± 3358816$^{++}$** | 8 | **16350000 ± 3063195$^{++}$** | 7 |
| Radiotherapy group | 760950 ± 575139 | 10 | 9543600 ± 4539824 | 5 | 8778950 ± 3114933 | 4 |
| Radiotherapy + cisplatin group | 766250 ± 515323 | 10 | 5188000 ± 2224171 | 6 | **6475000 ± 2292911*** | 4 7 |
| Kanglaite soft capsule group | 762580 ± 485051 | 10 | 9066700 ± 2236030 | 10 | 10584429 ± 3695704 | |
| Radiotherapy + Kanglaite soft capsule group | 774400 ± 476989 | 10 | 7340703 ± 2639117 | 8 | 8868667 ± 4666329 | 3 |
| Radiotherapy + GLP-3 low-dose group | 771970 ± 448693 | 10 | 8275244 ± 2176340 | 10 | **7076889 ± 2486348*** | 9 |
| Radiotherapy + GLP-3 high-dose group | 769960 ± 381742 | 10 | 7680260 ± 2102056 | 10 | **1892600 ± 724198*** | 6 |

Note: Compared to the normal control group, $^{++}P \leq 0.01$; compared to the model control group, $^{*}P \leq 0.05$, $^{**}P \leq 0.01$; compared to the radiotherapy group, $^{\#}P \leq 0.05$; compared to the radiotherapy + cisplatin group, $^{\&}P \leq 0.05$; compared to the Kanglaite soft capsule group, $^{*}P \leq 0.05$; compared to the radiotherapy + Kanglaite soft capsule group, $^{■}P \leq 0.05$.

**Effect of Ganoderma Lucidum Polysaccharide GLP-3 Combined with Radiotherapy on Organ Coefficients and Tumor Growth Inhibition Rates in Orthotopic H22-Bearing Mice**

As shown in Table 19, compared to the normal control group, the organ coefficients of the spleen and liver tissues in the model control group significantly increased ($P \leq 0.01$), and the thymus coefficient significantly decreased ($P \leq 0.01$). Compared to the model control group, the spleen organ coefficients in the radiotherapy + GLP-3 low-dose and high-dose groups, the Kanglaite soft capsule group, the cisplatin group, and the radiotherapy group significantly decreased ($P \leq 0.05$ or $P \leq 0.01$). The liver organ coefficients in the radiotherapy + GLP-3 low-dose group and the cisplatin group significantly decreased ($P \leq 0.05$ or $P \leq 0.01$). Compared to the radiotherapy group, the spleen organ coefficient in the Kanglaite soft capsule group significantly increased ($P \leq 0.05$ or $P \leq 0.01$). Compared to the radiotherapy + cisplatin group, the spleen organ coefficient in the Kanglaite soft capsule group significantly increased ($P \leq 0.05$ or $P \leq 0.01$). Compared to the Kanglaite soft capsule group, the spleen organ coefficients in radiotherapy + GLP-3 low-dose and high-dose groups significantly decreased ($P \leq 0.01$). There was no significant difference across the groups when compared to the radiotherapy + Kanglaite soft capsule group.

Table 19: Effect of Ganoderma lucidum polysaccharide GLP-3 combined with radiotherapy on organ coefficients in orthotopic H22-bearing mice (Mean ± SEM)

| Group | Spleen coefficient | Thymus coefficient | Liver coefficient |
|---|---|---|---|
| Normal control group | 0.325 ± 0.010 | 0.248 ± 0.028 | 4.836 ± 0.112 |
| Model control group | **1.131 ± 0.188$^{++}$** | **0.109 ± 0.026$^{++}$** | **15.449 ± 3.205$^{++}$** |
| Radiotherapy group | **0.499 ± 0.063**** | 0.118 ± 0.043 | 10.721 ± 1.378 |
| Radiotherapy + cisplatin group | **0.424 ± 0.140**** | 0.039 ± 0.017 | **7.962 ± 0.914*** |
| Kanglaite soft capsule group | **1.003 ± 0.110$^{\#\#\&\&}$** | 0.126 ± 0.042 | 14.943 ± 2.33 |
| Radiotherapy + Kanglaite soft capsule group | **0.618 ± 0.051*** | 0.226 ± 0.188 | 14.341 ± 2.991 |
| Radiotherapy + GLP-3 low-dose group | **0.539 ± 0.039****** | 0.088 ± 0.016 | **10.109 ± 1.135*** |

(continued)

| Group | Spleen coefficient | Thymus coefficient | Liver coefficient |
|---|---|---|---|
| Radiotherapy + GLP-3 high-dose group | **0.542 ± 0.083**$^{****}$ | 0.102 ± 0.025 | 13.685 ± 3.158 |

Note: Compared to normal control group, $^{+}P \leq 0.05$, $^{++}P \leq 0.01$; compared to model control group, $^{*}P \leq 0.05$, $^{**}P \leq 0.01$; compared to radiotherapy group, $^{\#}P \leq 0.05$; compared to radiotherapy + cisplatin group, $^{\&}P \leq 0.05$, $^{\&\&}P \leq 0.01$; compared to radiotherapy + Kanglaite soft capsule group, $^{■}P \leq 0.05$.

**Effect of Ganoderma Lucidum Polysaccharide GLP-3 Combined with Radiotherapy on Blood Biochemical Indicators in Orthotopic H22-Bearing Mice**

As shown in Table 20, compared to the normal control group, the blood levels of WBC, AST, BUN, and CRE in the model control group were significantly elevated ($P \leq 0.05$ or $P \leq 0.01$). Compared to the model control group, the blood levels of WBC, AST, BUN, and CRE in the radiotherapy + GLP-3 low-dose group, the cisplatin group, the Kanglaite soft capsule group, and radiotherapy group were significantly reduced ($P \leq 0.05$ or $P \leq 0.01$). In the radiotherapy + GLP-3 high-dose group, the blood levels of WBC, BUN, and CRE were significantly reduced ($P \leq 0.05$ or $P \leq 0.01$). In the Kanglaite soft capsule group, the blood levels of WBC and CRE were significantly reduced ($P \leq 0.05$ or $P \leq 0.01$). Compared to the radiotherapy group, the blood levels of CRE in the radiotherapy + GLP-3 low-dose and high-dose groups were significantly reduced ($P \leq 0.05$); the blood levels of WBC, AST, and BUN were significantly elevated ($P \leq 0.05$ or $P \leq 0.01$) and the CRE was significantly reduced ($P \leq 0.05$) in the Kanglaite soft capsule group. Compared to the radiotherapy + cisplatin group, the blood levels of WBC, AST, and BUN in the Kanglaite soft capsule group were significantly elevated ($P \leq 0.05$ or $P \leq 0.01$). Compared to the Kanglaite soft capsule group, the blood levels of AST and BUN in the radiotherapy + GLP-3 low-dose group were significantly reduced ($P \leq 0.05$ or $P \leq 0.01$); the blood levels of BUN in both the radiotherapy + GLP-3 high-dose group and the Kanglaite soft capsule group were significantly reduced ($P \leq 0.05$ or $P \leq 0.01$). No significant differences were observed in the other groups.

Table 20: Effect of Ganoderma lucidum polysaccharide GLP-3 combined with radiotherapy on blood biochemical indicators in orthotopic H22-bearing mice (Mean ± SEM)

| Group | WBC ($10^9$/L) | RBC ($10^{12}$/L) | ALT (U/L) | AST (U/L) | BUN (mmol/L) | CRE (umol/L) |
|---|---|---|---|---|---|---|
| Normal control group | **7.98 ± 0.66** | 9.54 ± 0.11 | 51.60 ± 2.85 | **103.30 ± 14.03** | 10.29 ± 0.41 | 34.17 ± 3.96 |
| Model control group | **10.47 ± 0.94**, | 9.41 ± 0 41 | 63.33 ± 8.72 | **656.67 ± 150.43**$^{++}$ | **13.58 ± 2.64**$^{+}$ | **46.92 ± 1.27**$^{++}$ |
| Radiotherapy group | **1.55 ± 039**$^{**}$ | 6.95 ± 121 | 6200 ± 1030 | **237.75 ± 85.33**$^{**}$ | **8.66 ± 0.83**$^{**}$ | **31.13 ± 5.85**$^{**}$ |
| Radiotherapy + cisplatin group | **0.57 ± 0.13**$^{**}$ | 6.79 ± 0.53 | 52.00 ± 8.69 | **230.00 ± 27.56**$^{**}$ | **9.00 ± 0.58**$^{*}$ | **23.53 ± 5.16**$^{**}$ |
| Kanglaite soft capsule group | **7.73 ± 0.8,3**$^{*\#\#\&\&}$ | 9.34 ± 0.18 | 76.00 ± 14.01 | **612.14 ± 136.83**$^{\#\#\&\&}$ | **14.06 ± 1.74**$^{\#\&}$ | **20.51 ± 2.56**$^{**\#}$ |
| Radiotherapy + Kanglaite soft capsule group | **2.07 ± 0.71**$^{**}$ | 8.75 ± 0.09 | 56.33 ± 9.67 | **351.33 ± 113.6-1**$^{*}$ | **9.42 ± 1.34**$^{***}$ | **25.13 ± 4.72**$^{**}$ |
| Radiotherapy + GLP-3 low-dose group | **1.88 ± 0.45**$^{**}$ | 8.54 ± 0.36 | 57.56 ± 6.62 | **337.61 ± 49.07**$^{****}$ | **8.54 ± 0.49**$^{****}$ | **18.57 ± 2.22**$^{**\#}$ |
| Radiotherapy + GLP-3 lugh-dose group | **2.49 ± 0.73**$^{**}$ | 8.44 ± 0.36 | 71.33 ± 22.46 | 468.33 ± 105.82 | **8.50 ± 0.74**$^{****}$ | **20.02 ± 2.61**$^{**\#}$ |

Note: Compared to normal control group, $^{+}P \leq 0.05$, $^{++}P \leq 0.01$; compared to model control group, $^{*}P \leq 0.05$, $^{**}P \leq 0.01$; compared to radiotherapy group, $^{\#}P \leq 0.05$, $^{\#\#}P \leq 0.01$; compared to radiotherapy + cisplatin group, $^{\&}P \leq 0.05$, $^{\&\&}P \leq 0.01$; compared to Kanglaite soft capsule group, $^{*}P < 0.05$, $^{**}P \leq 0.01$.

**Effect of Ganoderma Lucidum Polysaccharide GLP-3 Combined with Radiotherapy on Blood $CD3^+/CD4^+$ and $CD3^+/CD8^+$ Ratios in Orthotopic H22-Bearing Mice**

As shown in Table 21, compared to the normal control group, there was a trend of increased blood $CD3^+/CD4^+$ and $CD3^+/CD8^+$ ratios in the model control group of mice, though the differences were not significant. There were no significant differences between the treatment groups and the model control group. Compared to the radiotherapy + cisplatin group, significant reductions in $CD3^+/CD8^+$ ratios were observed in the radiotherapy + GLP-3 low-dose group and the Kanglaite soft capsule group ($P \leq 0.05$ or $P \leq 0.01$). No significant differences were observed in the other groups.

Table 21: Effect of Ganoderma lucidum polysaccharide GLP-3 combined with radiotherapy on blood $CD3^+/CD4^+$ and $CD3^+/CD8^+$ ratios in orthotopic H22-bearing mice (Mean ± SEM)

| Group | $CD3^+/CD4^+$ | $CD3^+/CD8^+$ |
|---|---|---|
| Normal control group | 9.18 ± 1.08 | 16.84 ± 2.46 |
| Model control group | 19.35 ± 13.42 | 42.75 ± 32.55 |
| Radiotherapy group | 14.10 ± 3.24 | 52.76 ± 19.06 |
| Radiotherapy + cisplatin group | 23.05 ± 12.70 | 126.56 ± 67.07 |
| Kanglaite soft capsule group | 5.22 ± 1.19 | **10.38 ± 2.78**$^{\&\&}$ |
| Radiotherapy + Kanglaite soft capsule group | 20.18 ± 4.75 | **65.34 ± 27.66**$^{\&}$ |
| Radiotherapy + GLP-3 low-dose group | 21.39 ± 3.35 | **46.40 ± 14.51**$^{\&\&}$ |
| Radiotherapy + GLP-3 high-dose group | 23.07 ± 12.23 | 61.77 ± 1.77 |

Note: Compared to the model control group, $^*P \leq 0.05$; compared to the radiotherapy + cisplatin group, $^{\&}P \leq 0.05$, $^{\&\&}P \leq 0.01$.

As shown in Figures 41, 42, and 43, the mice in the model control group exhibited extensive infiltration and necrosis of H22 cells in the liver, increased number of hepatic sinusoidal cells, and infiltration of inflammatory cells; noticeable extramedullary hematopoiesis and diffuse red pulp in the spleen, and infiltration of liver cancer cells in the thymus with a reduced number of cortical/medullary lymphocytes. In the radiotherapy group and the radiotherapy + cisplatin group, extensive H22 cell infiltration and necrosis were observed along with increased extramedullary hematopoiesis in the spleen and a decreased number of white pulp cells, with a reduction in thymic lymphocyte counts. After administration of Ganoderma lucidum polysaccharide GLP-3 combined with radiotherapy, each group showed reduced liver cancer cell infiltration and necrosis, increased spleen extramedullary hematopoiesis, increased number of plasma cells in the white pulp, and alleviated thymic pathology.

As shown in Figures 41, 42, and 43, the corresponding groups are as follows: A: normal group, B: model control group, C: cisplatin group, D: Kanglaite soft capsule group, E: cisplatin + Kanglaite soft capsule group, F: cisplatin + GLP-3 low-dose group, G: cisplatin + GLP-3 high-dose group

Conclusion

Ganoderma lucidum polysaccharide GLP-3 combined with radiotherapy significantly inhibits the growth of tumors in orthotopic H22-bearing mice and demonstrates a notable synergistic effect.

**Discussion and Conclusion**

Liver cancer is a highly lethal malignancy, where current treatments involving surgical resection, chemotherapy, and radiotherapy merely delay symptoms, making complete cure extremely difficult. Liver cancer is notably resistant to chemotherapy, especially in cases of advanced liver cancer, where there is no reliable evidence proving that systemic chemotherapy can improve overall survival of patients with advanced liver cancer.

The results of this study showed that in the model control group of mice, tumor volume significantly increased, spleen and liver indices significantly increased, red blood cell counts in the blood significantly increased, white blood cell counts significantly decreased, and liver and kidney functions were significantly abnormal. These findings indicate a decline in lymphatic system function during tumor progression in the model control group of mice. After the last dose, Ganoderma lucidum polysaccharide GLP-3 combined with radiotherapy was able to significantly inhibit tumor growth in mice, significantly lower thymus and spleen indices, significantly reduce red blood cell counts, and significantly decrease liver and kidney function indicators. $CD4^+$T cells and $CD8^+$T cells mediate tumor immune responses, where $CD8^+$T cells are the main effector cells of tumor immunity. Results indicate that Ganoderma lucidum polysaccharide GLP-3 combined

with radiotherapy significantly lowered $CD3^+/CD4^+$ and $CD3^+/CD8^+$ ratios in the mice's blood, suggesting that Ganoderma lucidum polysaccharide GLP-3 combined with radiotherapy can protect immune organs and enhance the body's own immune function, thereby playing a role in reducing toxicity and enhancing antitumor effects. Histopathological results also showed that Ganoderma lucidum polysaccharide GLP-3 can enhance the body's immunity. Additionally, when compared to Kanglaite soft capsule combined with radiotherapy, Ganoderma lucidum polysaccharide GLP-3 combined with radiotherapy significantly reduced tumor, spleen indices, and thymus indices, indicating that Ganoderma lucidum polysaccharide GLP-3 combined with radiotherapy has a stronger antitumor effect than Kanglaite soft capsule combined with radiotherapy.

In conclusion, Ganoderma lucidum polysaccharide GLP-3 combined with radiotherapy significantly inhibits the growth of tumors in orthotopic H22-bearing mice and demonstrates a notable synergistic effect.

The foregoing description concerns merely exemplary embodiments of the present invention and is not intended to limit the invention. Any modifications, equivalent substitutions, and improvements made within the spirit and principles of the invention should be included within the scope of the invention's protection.

## Claims

1. A type of Ganoderma lucidum polysaccharide GLP-3, **characterized in that** the molecular structural formula of the Ganoderma lucidum polysaccharide GLP-3 is

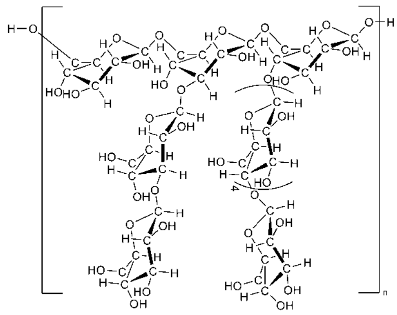

;

the molecular formula is: $(C_{66}H_{110}O_{55})_n$, where n = 30-46.

2. The Ganoderma lucidum polysaccharide GLP-3 according to claim 1, wherein n is selected from 30, 31, 32, 33, 34, 35, 36, 37, 38, 39, 40, 41, 42, 43, 44, 45, or 46.

3. A method for extracting the Ganoderma lucidum polysaccharide GLP-3, comprising the steps of:

S1. dusting and drying Ganoderma lucidum, then crushing Ganoderma lucidum to make Ganoderma lucidum powder;
S2. placing the crushed Ganoderma lucidum powder in a sealed container mixed with water, heating under high temperature and pressure to fully dissolve the Ganoderma lucidum powder with the water into a medicinal juice solution;
S3. using membrane concentration technology to separate the medicinal juice solution to obtain a concentrated solution with an active ingredient and not fully dissolved medicinal residue; and
S4. mixing the concentrated solution containing the active ingredient with pure water to a water solution with a preset concentration, followed by multiple column chromatography separations as well as concentration and lyophilization to obtain the Ganoderma lucidum polysaccharide GLP-3 with the active ingredient.

4. The method for extracting the Ganoderma lucidum polysaccharide GLP-3 according to claim 3, **characterized in that** in step S2, the Ganoderma lucidum powder is thoroughly mixed with the water and heated to 105-200°C, and the boiling time lasts for 2-6 h, during which the Ganoderma lucidum powder and the water in the sealed container are fully dissolved under high temperature and pressure into a mixed medicinal liquid.

5. The method for extracting the Ganoderma lucidum polysaccharide GLP-3 according to claim 3, **characterized in that** in step S2, the Ganoderma lucidum powder is thoroughly mixed with the water and heated to 105-170°C, and the boiling time lasts for 3-6 h, during which the Ganoderma lucidum powder and the water in the sealed container are fully

dissolved under high temperature and pressure into a mixed medicinal liquid.

6. The method for extracting the Ganoderma lucidum polysaccharide GLP-3 according to claim 4 or 5, **characterized in that** in step S4, the concentrated liquid containing the active ingredient is mixed with the pure water at a concentration ratio of 1:2 to 1:5.

7. The method for extracting the Ganoderma lucidum polysaccharide GLP-3 according to claim 6, **characterized in that** in step S3, Ganoderma lucidum residue is removed from the extracted water solution containing the active ingredient by using the membrane concentration technology, obtaining a concentrated liquid or paste containing the active ingredient.

8. The method for extracting the Ganoderma lucidum polysaccharide GLP-3 according to claim 7, **characterized in that** in step S1, the Ganoderma lucidum is rinsed with clean water to remove surface dust, then dried at 105°C in drying equipment, and after drying, the Ganoderma lucidum is crushed in crushing equipment to obtain the Ganoderma lucidum powder, with a particle size larger than 60 mesh.

9. The method for extracting the Ganoderma lucidum polysaccharide GLP-3 according to claim 8, **characterized in that** in step S2, the mixed liquid in the sealed container is heated to temperatures of 105°C, 110°C, 115°C, 120°C, 125°C, 130°C, 135°C, 140°C, 145°C, 150°C, 155°C, 160°C, 165°C, or 170°C, with boiling times of 3 h, 3.5 h, 4 h, 4.5 h, 5 h, 5.5 h, or 6 h, during which the Ganoderma lucidum powder in the sealed container is fully dissolved with the water into the mixed medicinal liquid in the high temperature and pressure environment.

10. The application of the Ganoderma lucidum polysaccharide GLP-3 and cisplatin in the preparation of anti-lung cancer and liver cancer drug products according to claim 1 or 2, **characterized by** the combined use of the Ganoderma lucidum polysaccharide GLP-3 and the chemotherapy drug cisplatin, which can alleviate the toxic side effects caused by chemotherapy drugs on the human body, control and reduce tumor masses, and reduce and eliminate cancer cells.

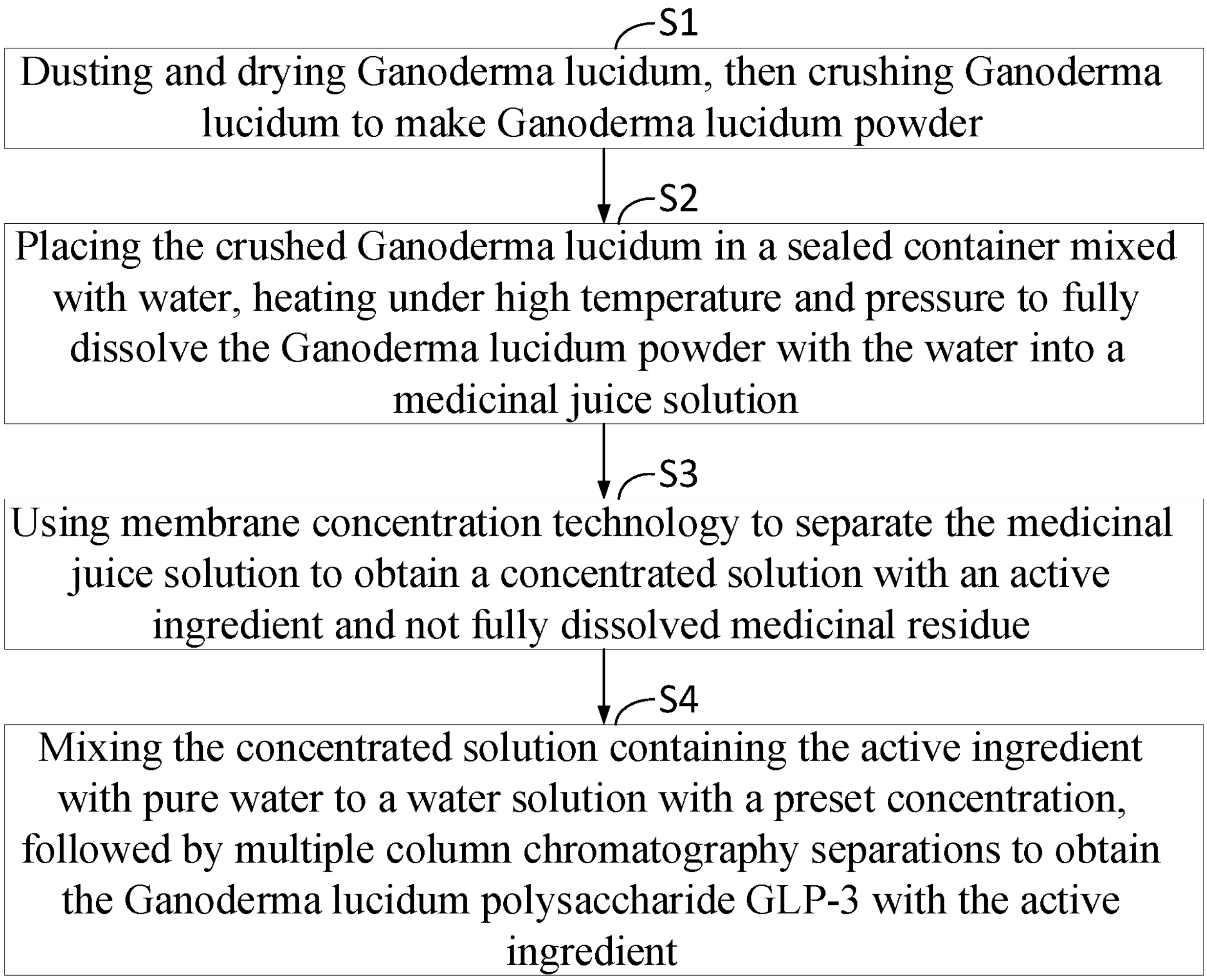
S1
Dusting and drying Ganoderma lucidum, then crushing Ganoderma lucidum to make Ganoderma lucidum powder
S2
Placing the crushed Ganoderma lucidum in a sealed container mixed with water, heating under high temperature and pressure to fully dissolve the Ganoderma lucidum powder with the water into a medicinal juice solution
S3
Using membrane concentration technology to separate the medicinal juice solution to obtain a concentrated solution with an active ingredient and not fully dissolved medicinal residue
S4
Mixing the concentrated solution containing the active ingredient with pure water to a water solution with a preset concentration, followed by multiple column chromatography separations to obtain the Ganoderma lucidum polysaccharide GLP-3 with the active ingredient

Fig. 1

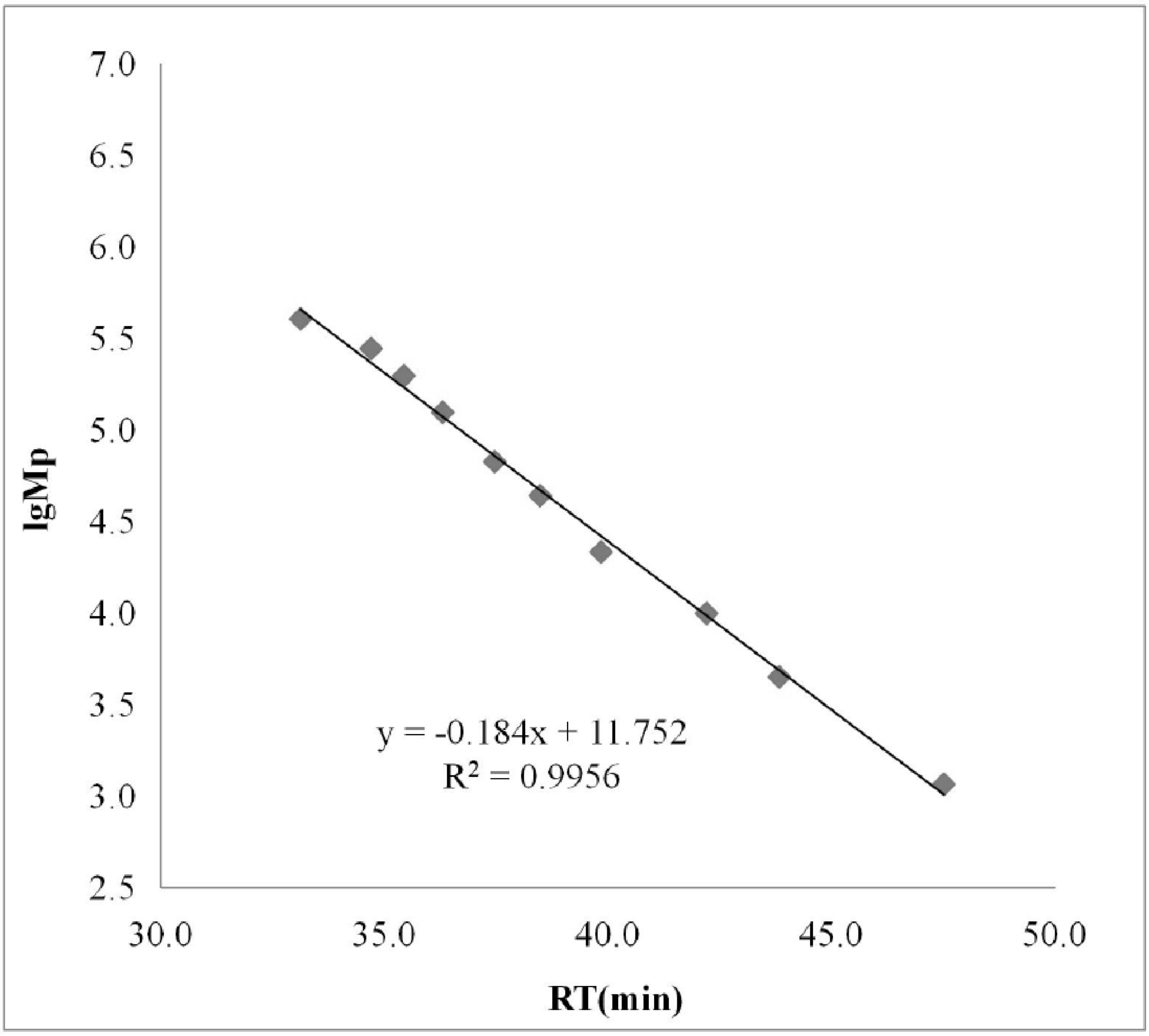
7.0
6.5
6.0
5.5
5.0
4.5
4.0
3.5
3.0
2.5
lgMp
y = -0.184x + 11.752
R² = 0.9956
30.0
35.0
40.0
45.0
50.0
RT(min)

Fig. 2

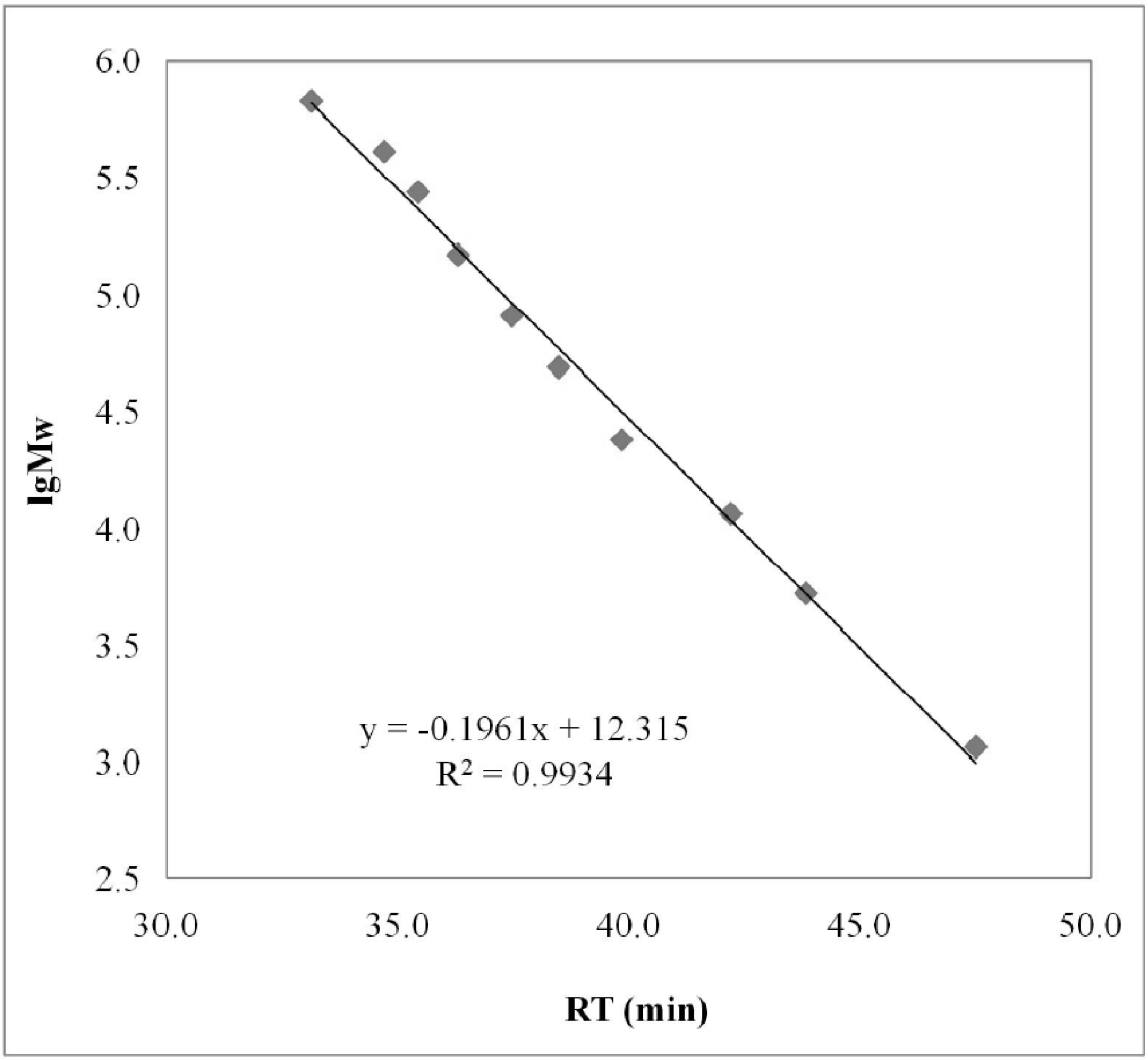
6.0
5.5
5.0
4.5
4.0
3.5
3.0
2.5
lgMw
y = -0.1961x + 12.315
R² = 0.9934
30.0
35.0
40.0
45.0
50.0
RT (min)

Fig. 3

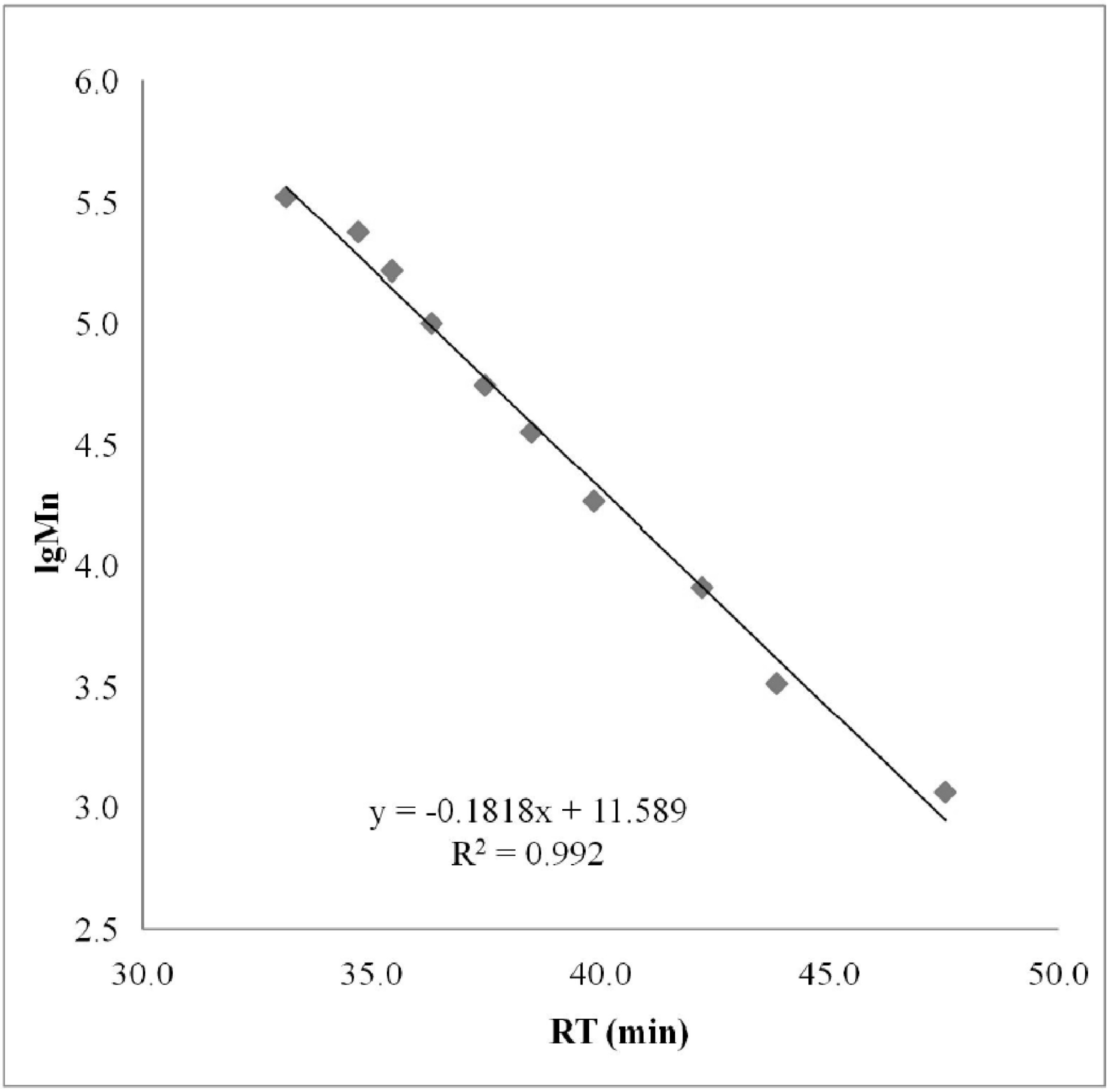
6.0
5.5
5.0
4.5
4.0
3.5
3.0
2.5
lgMn
y = -0.1818x + 11.589
R² = 0.992
30.0
35.0
40.0
45.0
50.0
RT (min)

Fig. 4

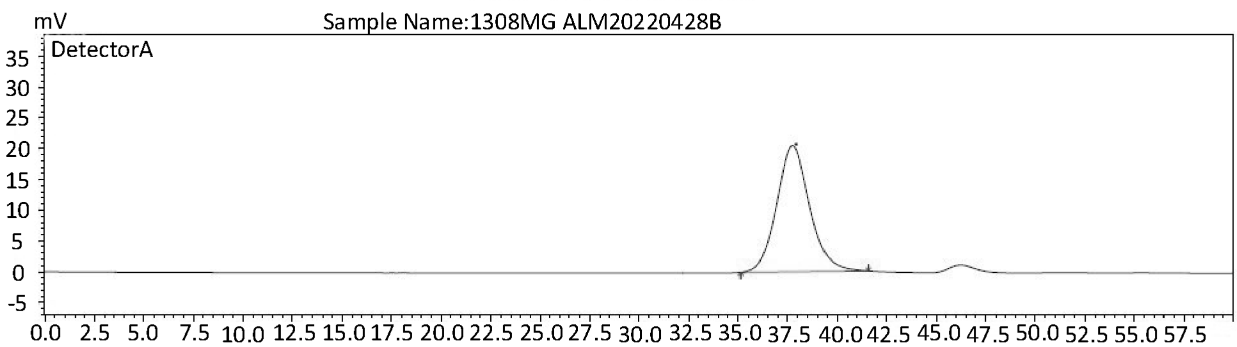
Data file Name:1308MG ALM2022B.lcd
Sample Name:1308MG ALM20220428B
mV
DetectorA
35
30
25
20
15
10
5
0
-5
0.0 2.5 5.0 7.5 10.0 12.5 15.0 17.5 20.0 22.5 25.0 27.5 30.0 32.5 35.0 37.5 40.0 42.5 45.0 47.5 50.0 52.5 55.0 57.5

Fig. 5

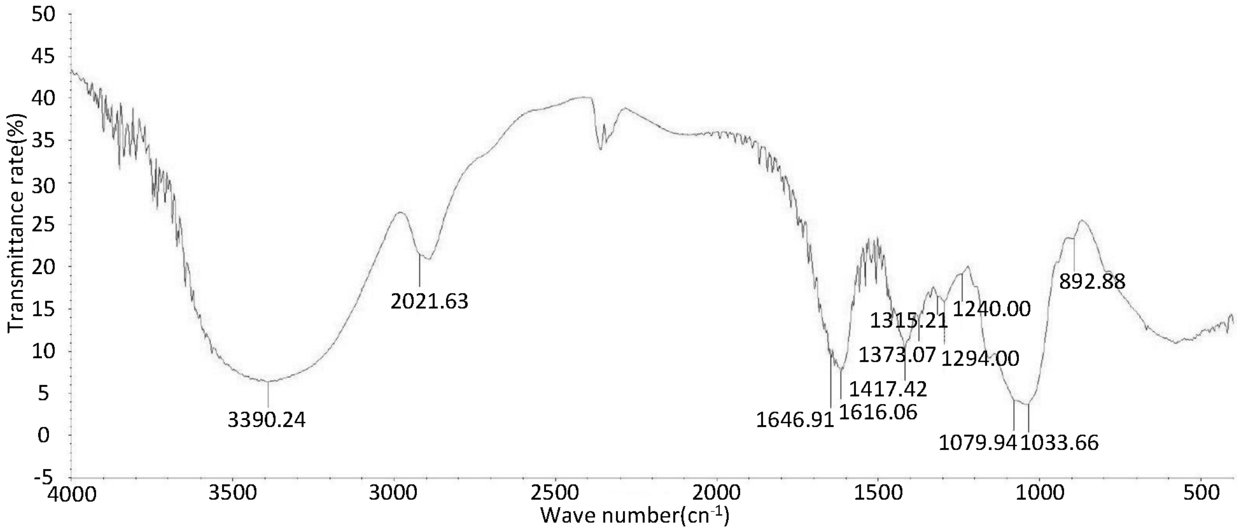
Transmittance rate(%)
Wave number(cn-1)
3390.24
2021.63
1646.91
1616.06
1417.42
1373.07
1315.21
1294.00
1240.00
1079.94
1033.66
892.88

Fig. 6

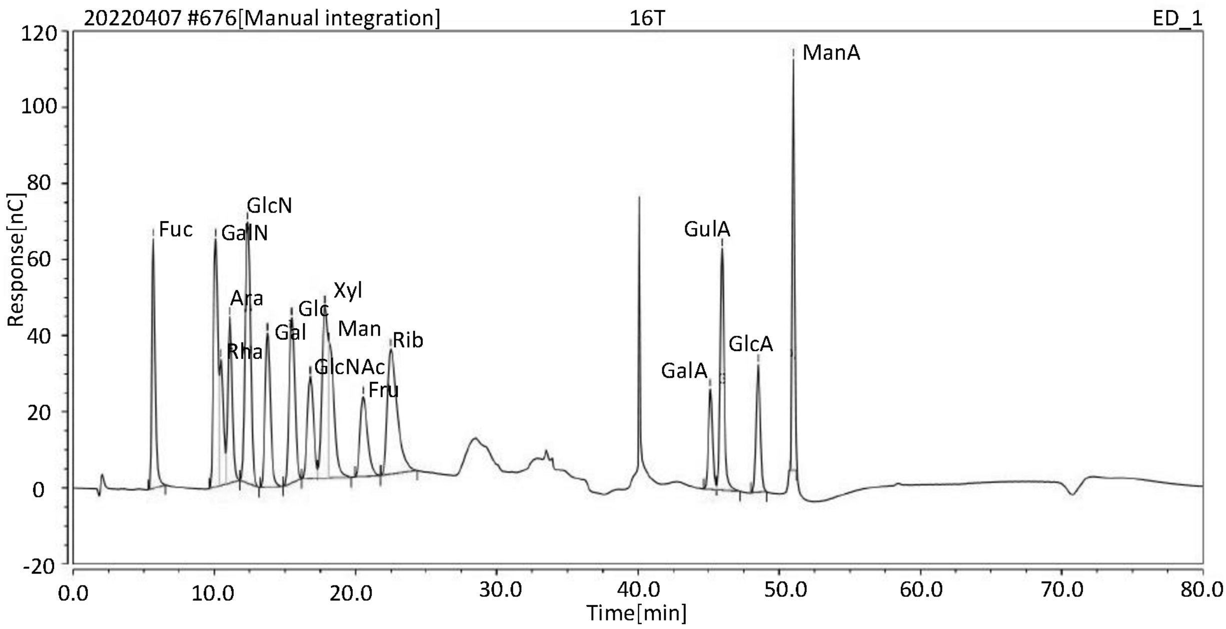
20220407 #676[Manual integration]
16T
ED_1
Response[nC]
Time[min]
Fuc
GalN
GlcN
Ara
Rha
Gal
Glc
Xyl
Man
GlcNAc
Fru
Rib
GulA
GalA
GlcA
ManA

Fig. 7

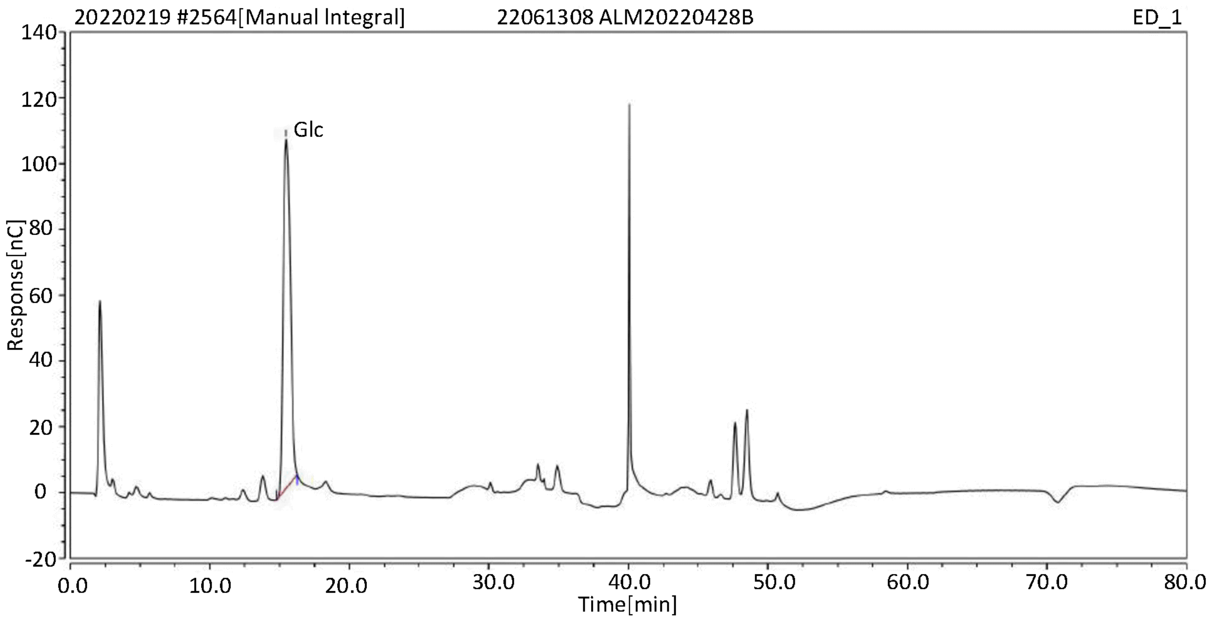

20220219 #2564[Manual Integral]
22061308 ALM20220428B
ED_1
Glc
Response[nC]
Time[min]
140
120
100
80
60
40
20
0
-20
0.0
10.0
20.0
30.0
40.0
50.0
60.0
70.0
80.0


Fig. 8

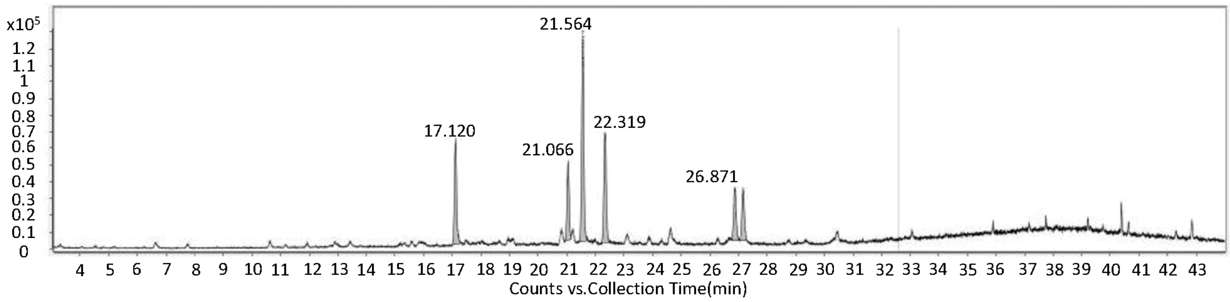

x10^5
21.564
17.120
21.066
22.319
26.871
Counts vs.Collection Time(min)


Fig. 9

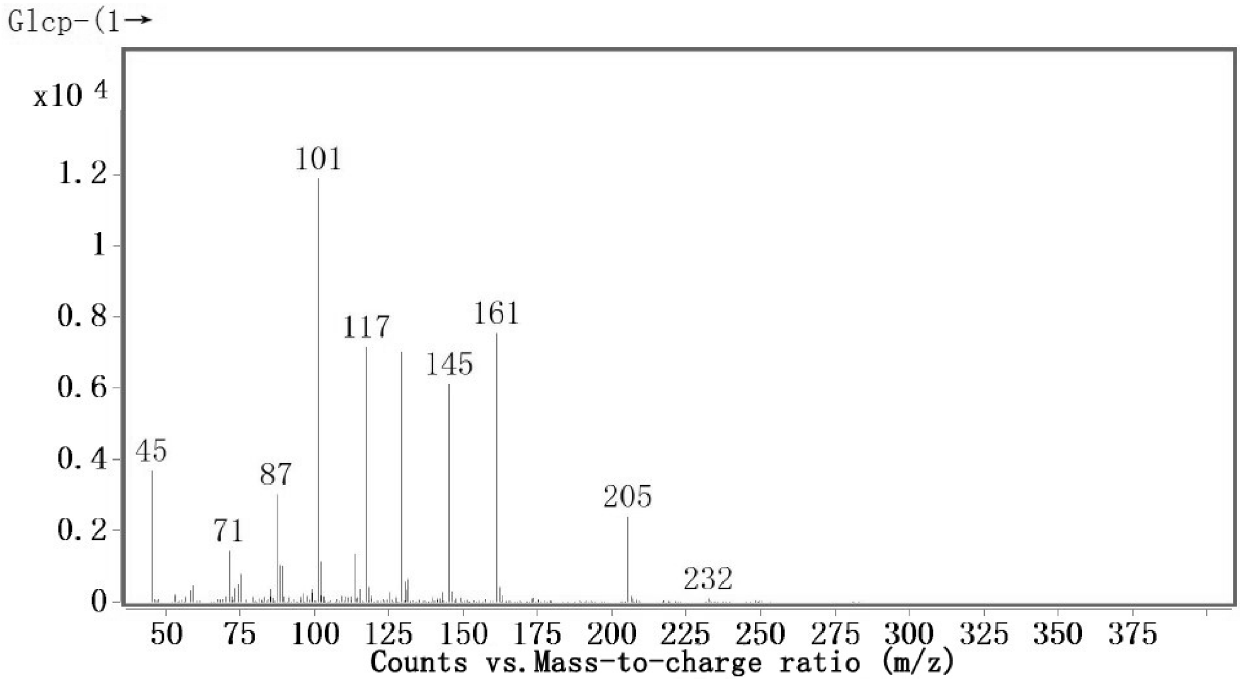
Glcp-(1→
x10 4
1.2
1
0.8
0.6
0.4
0.2
0
101
117
161
145
45
87
71
205
232
50 75 100 125 150 175 200 225 250 275 300 325 350 375
Counts vs.Mass-to-charge ratio (m/z)

Fig. 10

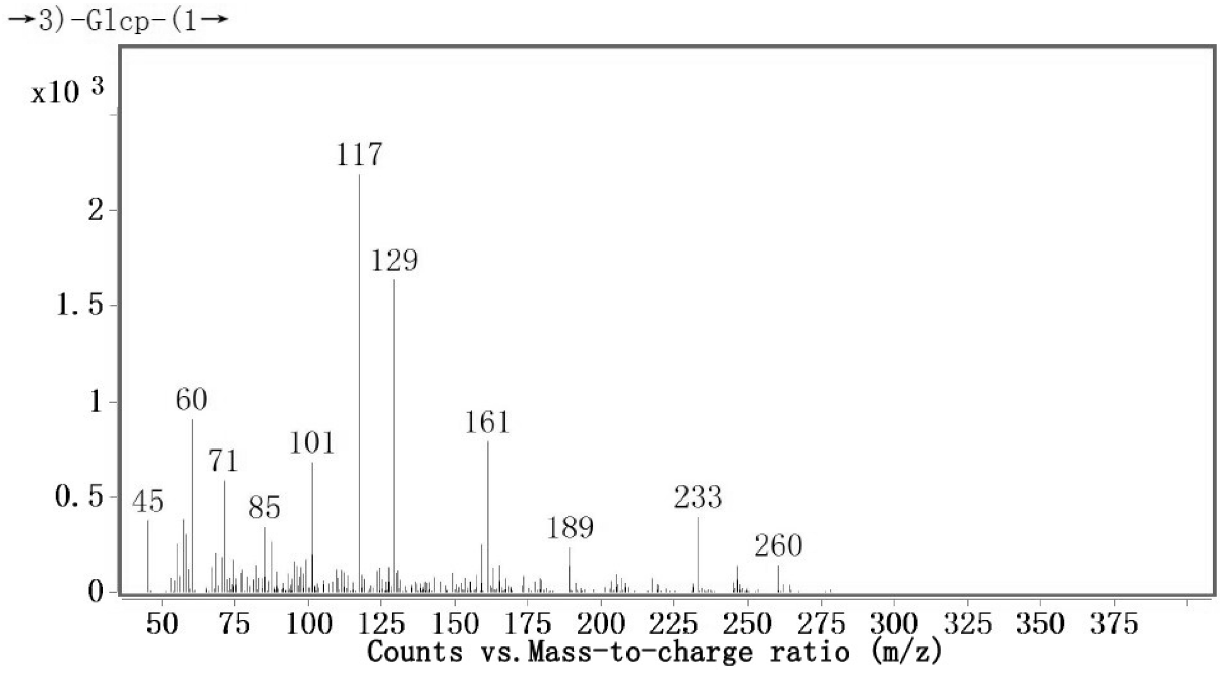
→3)-Glcp-(1→
x10 3
2
1.5
1
0.5
0
117
129
60
161
101
71
45
85
189
233
260
50 75 100 125 150 175 200 225 250 275 300 325 350 375
Counts vs.Mass-to-charge ratio (m/z)

Fig. 11

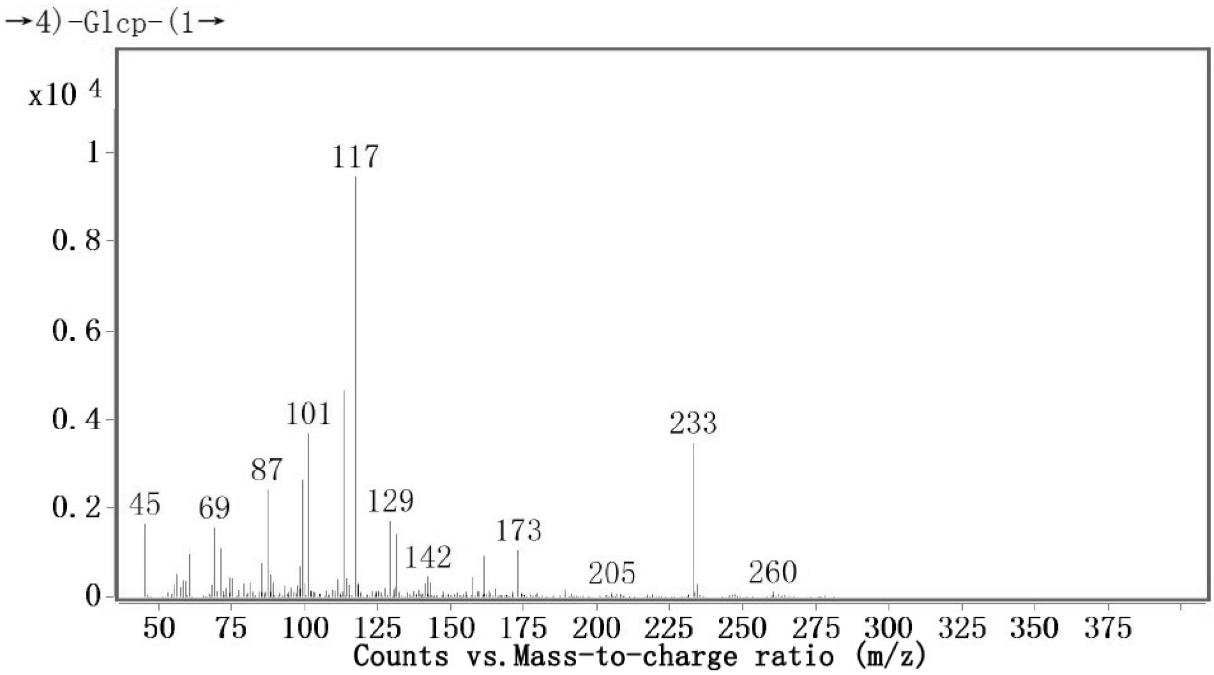
→4)-Glcp-(1→
x10 4
1
0.8
0.6
0.4
0.2
0
117
101
233
87
45
69
129
173
142
205
260
50 75 100 125 150 175 200 225 250 275 300 325 350 375
Counts vs.Mass-to-charge ratio (m/z)

Fig. 12

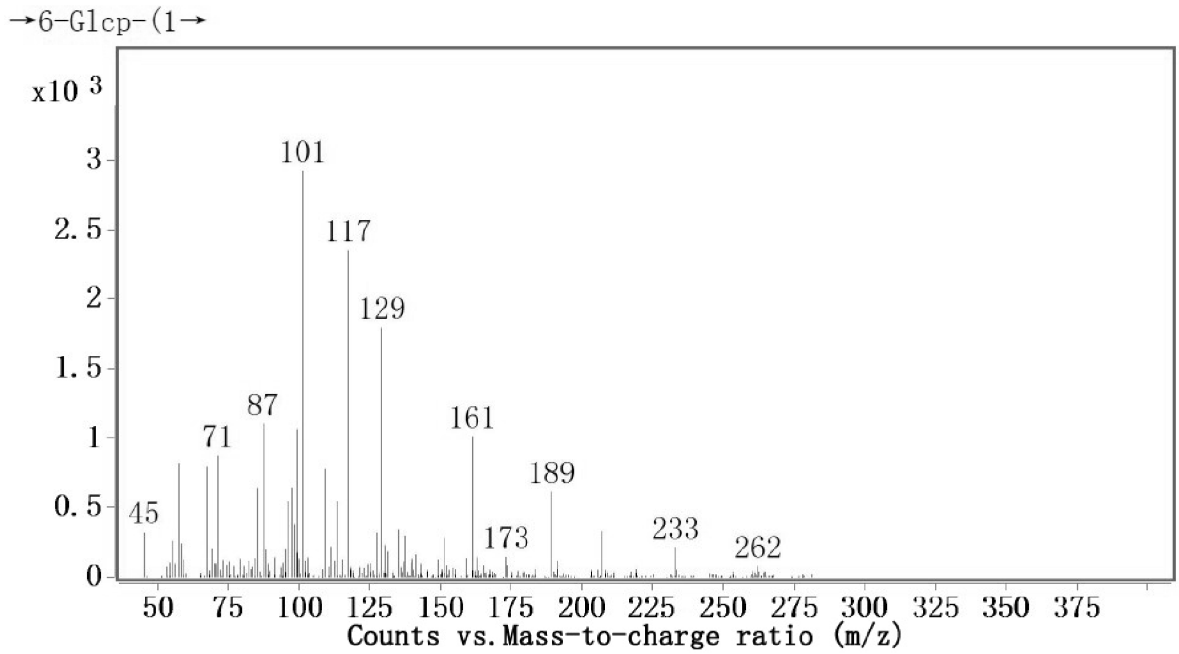
→6-Glcp-(1→
x10 3
3
2.5
2
1.5
1
0.5
0
101
117
129
87
71
161
189
45
173
233
262
50 75 100 125 150 175 200 225 250 275 300 325 350 375
Counts vs.Mass-to-charge ratio (m/z)

Fig. 13

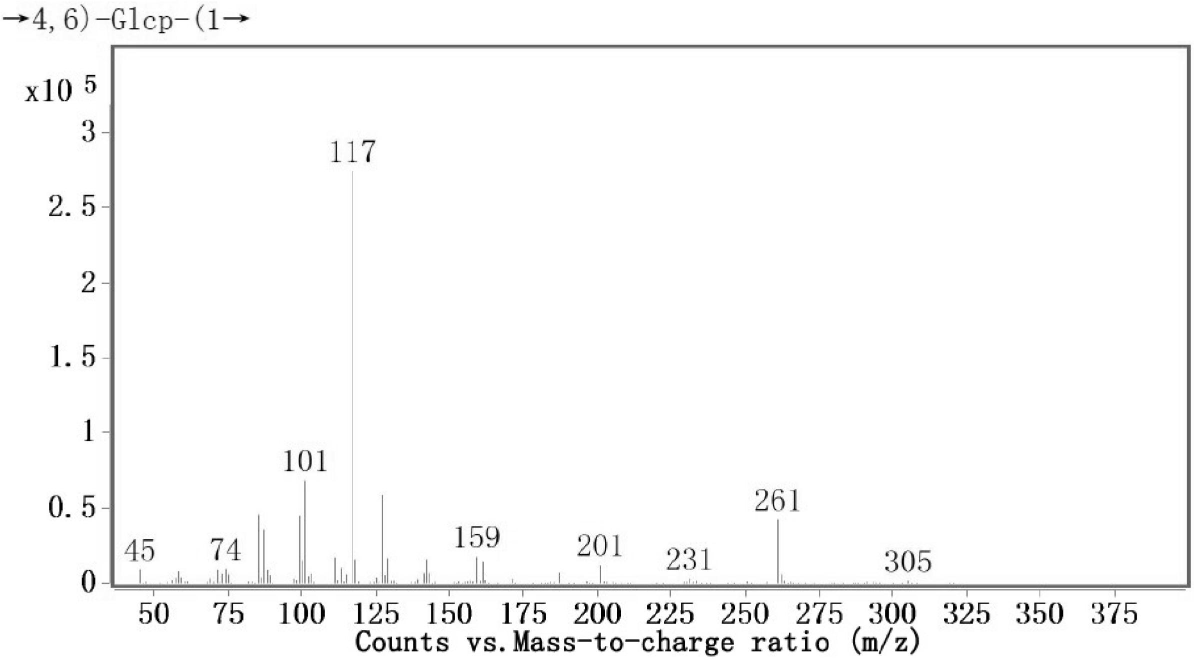
→4, 6)-Glcp-(1→
x10 5
3
2.5
2
1.5
1
0.5
0
117
101
45
74
159
201
231
261
305
50 75 100 125 150 175 200 225 250 275 300 325 350 375
Counts vs. Mass-to-charge ratio (m/z)

Fig. 14

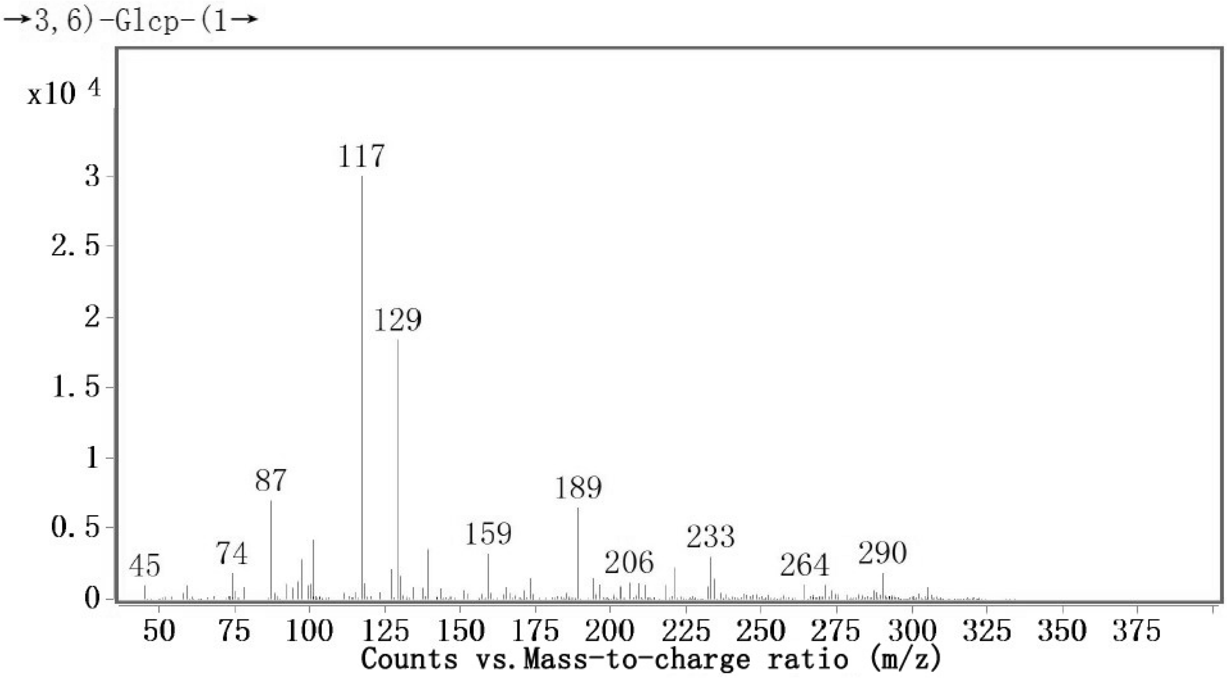
→3, 6)-Glcp-(1→
x10 4
3
2.5
2
1.5
1
0.5
0
117
129
87
189
45
74
159
206
233
264
290
50 75 100 125 150 175 200 225 250 275 300 325 350 375
Counts vs. Mass-to-charge ratio (m/z)

Fig. 15

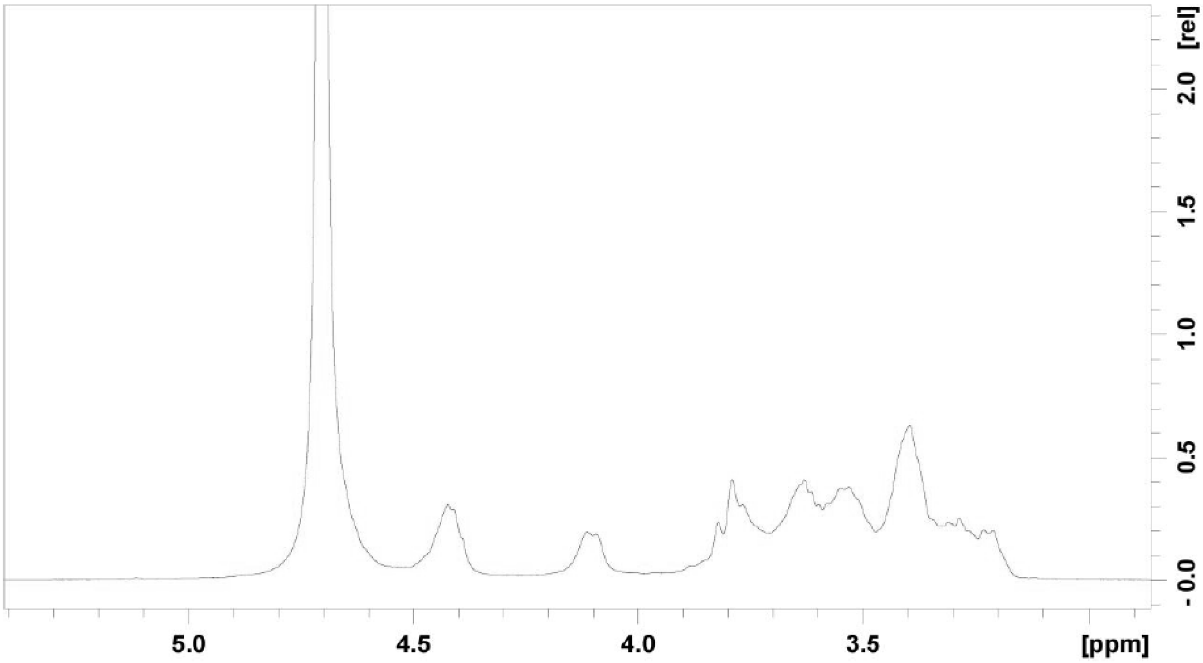
[rel]
2.0
1.5
1.0
0.5
- 0.0
5.0
4.5
4.0
3.5
[ppm]

Fig. 16

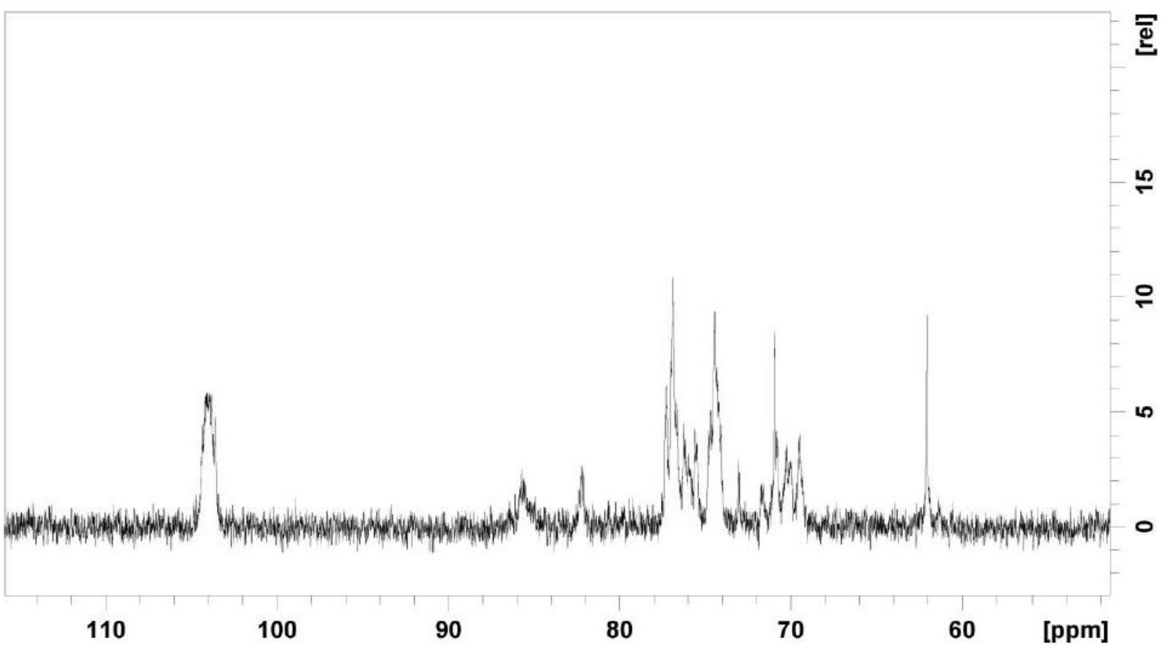
[rel]
15
10
5
0
110
100
90
80
70
60
[ppm]

Fig. 17

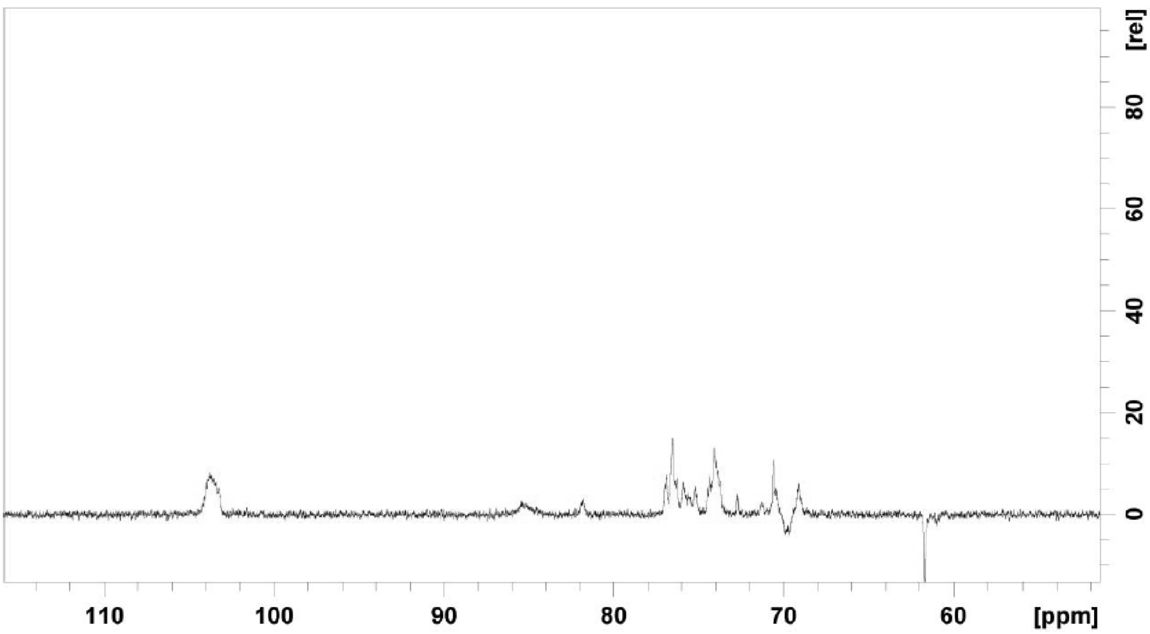


Fig. 18

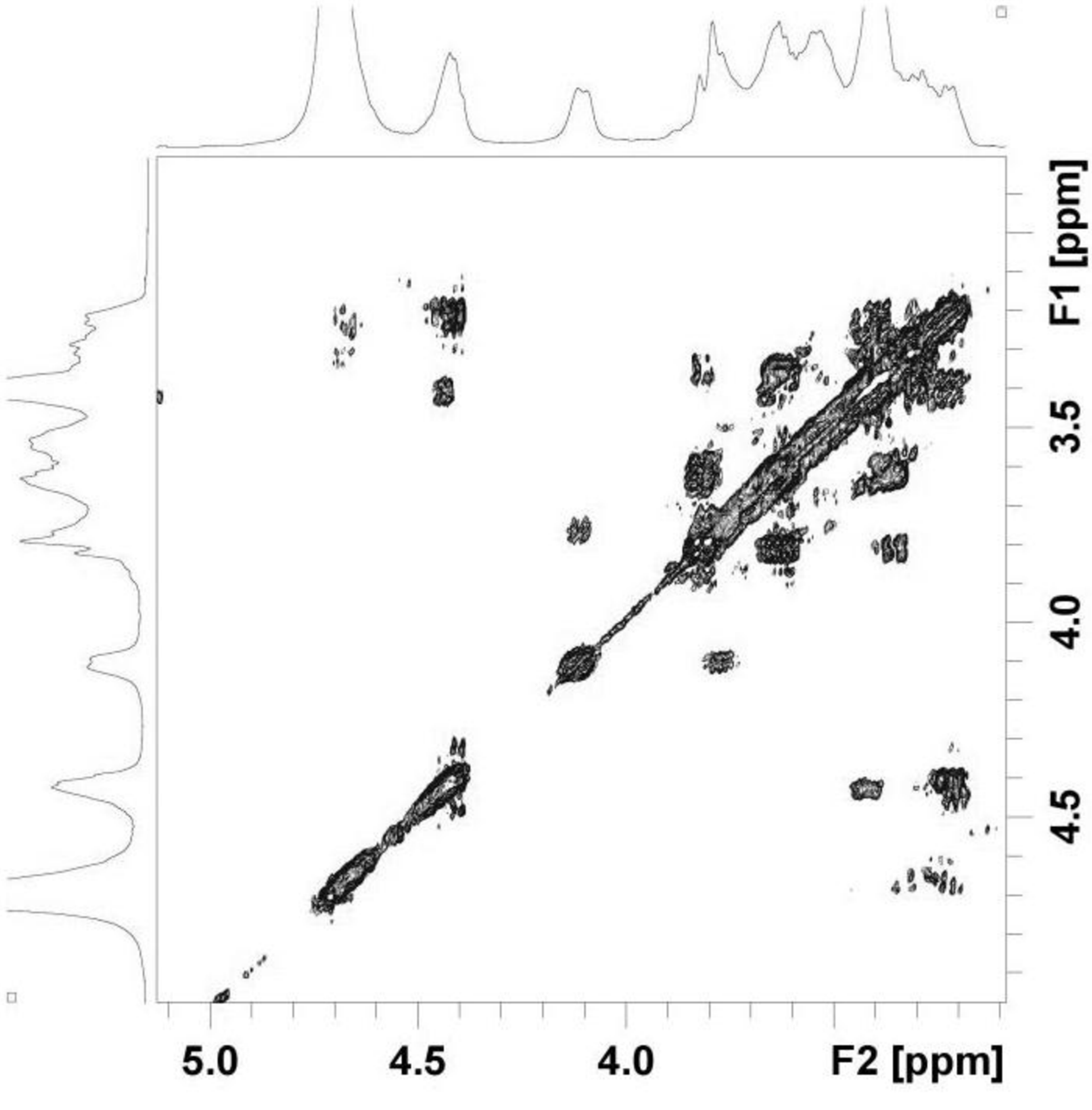


Fig. 19

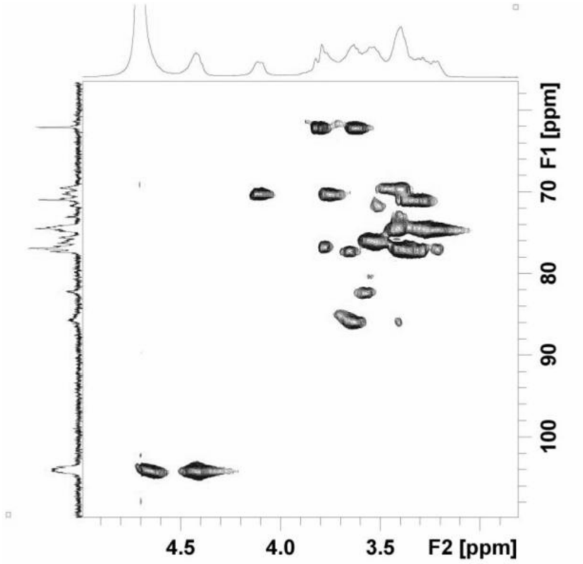


Figure 20

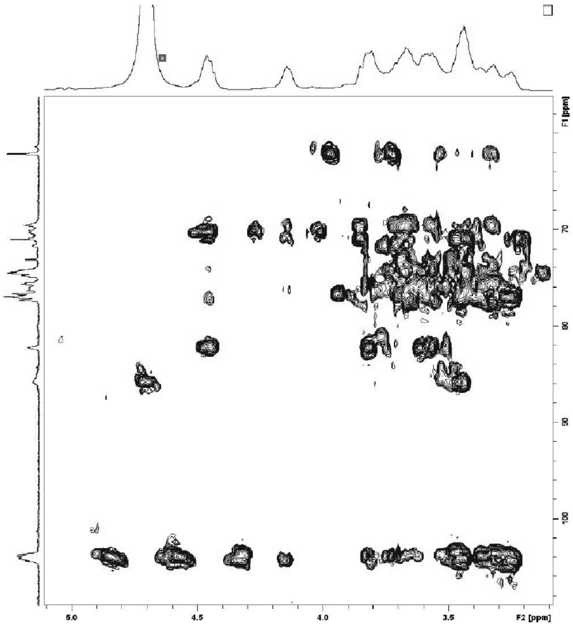

Figure 21

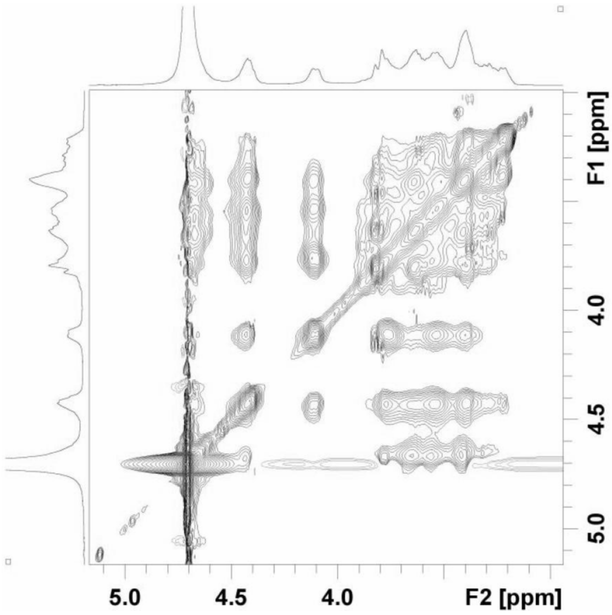


Figure 22

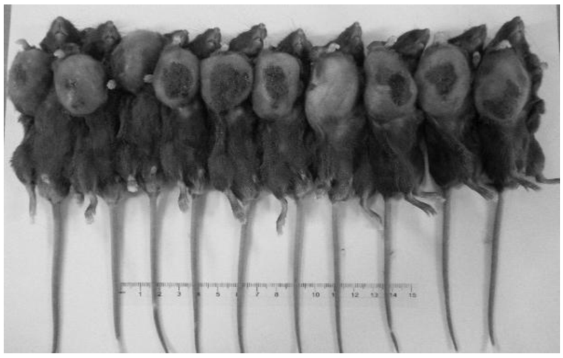

Figure 23

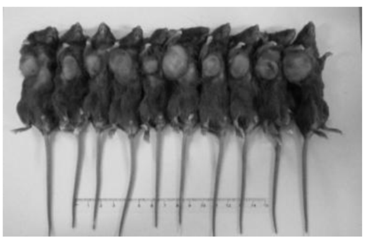

Figure 24

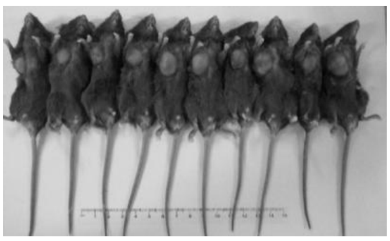

Figure 25

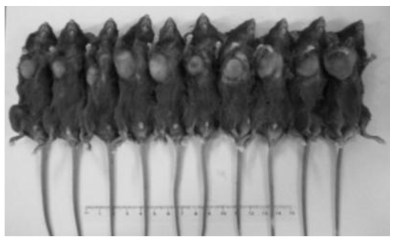

Figure 26

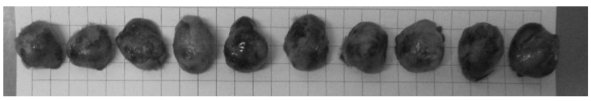

Figure 27

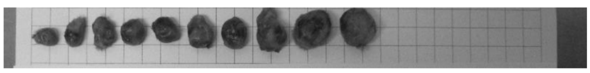

Figure 28

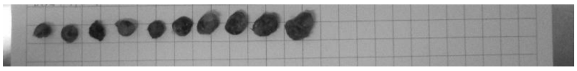
Figure 29

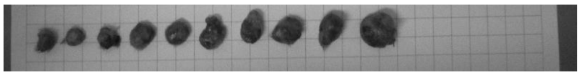
Figure 30

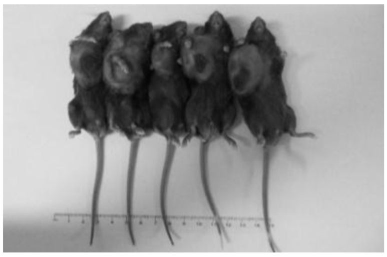
Figure 31

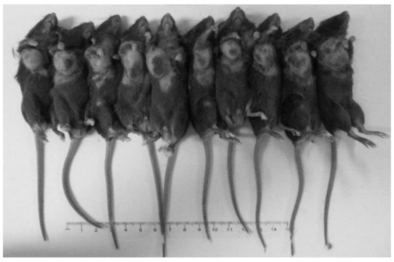
Figure 32

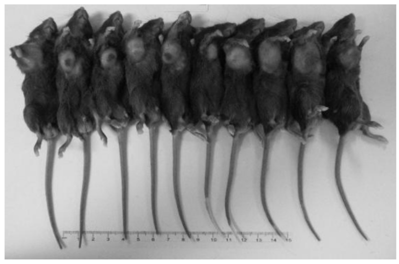
Figure 33

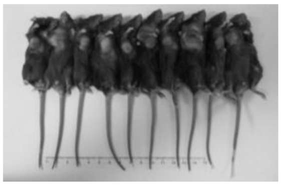
Figure 34

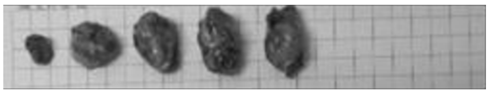
Figure 35

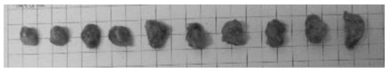
Figure 36

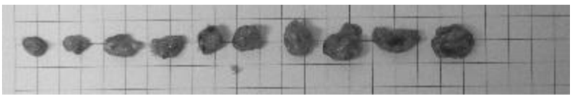
Figure 37

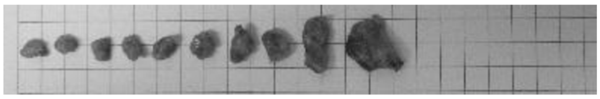
Figure 38

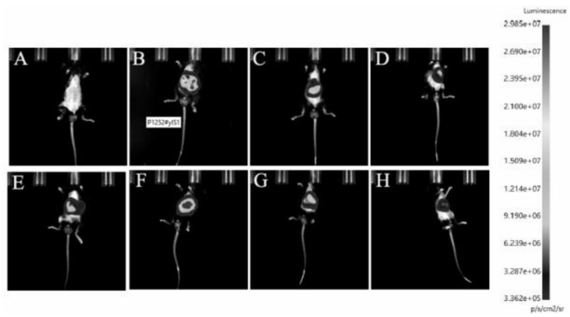

Figure 39

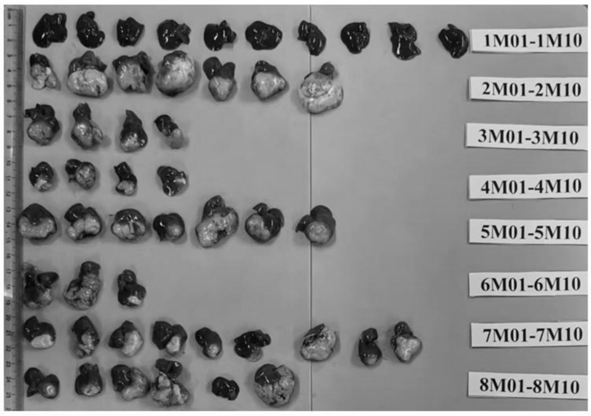

Figure 40

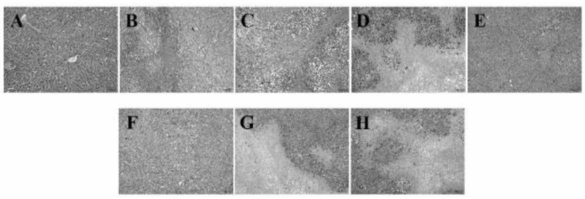

Figure 41

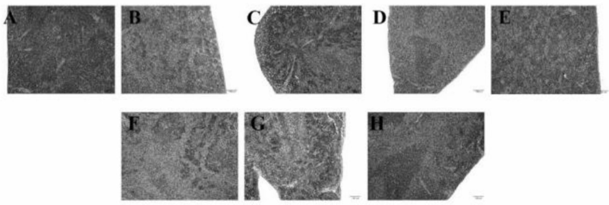

Figure 42

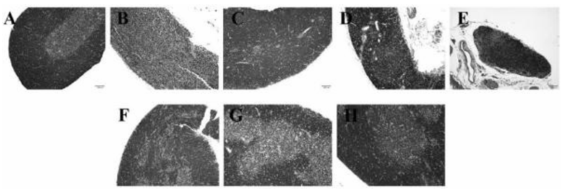

Figure 43

**INTERNATIONAL SEARCH REPORT**

International application No.
**PCT/CN2024/080445**

**A. CLASSIFICATION OF SUBJECT MATTER**

C08B37/00(2006.01)i; A61K31/715(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C08B A61K A61P

Documentation searched other than minimum documentation to the extent that such documents are included in the fields searched

Electronic data base consulted during the international search (name of data base and, where practicable, search terms used)

CNKI, 万方, WANFANG, CJFD, CNTXT, ENTXT, DWPI, STN, ISI Web of Science: 多糖, 高温, 高压, 灵芝, 柱层析, 色谱柱, 膜浓缩, 抗肿瘤, 癌, column, chromatography, ganoderma, lucidum, polysaccharide, pressure, high, temperature, membrane, concentration, cancer

**C. DOCUMENTS CONSIDERED TO BE RELEVANT**

| Category* | Citation of document, with indication, where appropriate, of the relevant passages | Relevant to claim No. |
|---|---|---|
| A | 操丽丽等 (CAO, Lili et al.). "高压热水提取灵芝多糖及对其抗氧化活性的影响 (Study on Extraction of Polysaccharides from Ganoderma lucidum by Hot Compressed Water and Its Antioxidant Activities)" *食品科学技术学报 (Journal of Food Science and Technology),* Vol. 36, No. 2, 17 April 2018 (2018-04-17), pages 58-62<br>section 1.5, and table 1 | 1-10 |
| A | CN 108976314 A (OCEAN UNIVERSITY OF CHINA) 11 December 2018 (2018-12-11)<br>entire document | 1-10 |
| A | CN 108250320 A (YANGLING DAILYHEALTH BIO-ENGINEERING TECHNOLOGY CO., LTD.) 06 July 2018 (2018-07-06)<br>entire document | 1-10 |
| A | CN 107857828 A (DAXINGANLING LINGOBERRY BOREAL BIOTECH CO., LTD.) 30 March 2018 (2018-03-30)<br>entire document | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 June 2024** | **07 June 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/080445

C. DOCUMENTS CONSIDERED TO BE RELEVANT

| Category* | Citation of document, with indication, where appropriate, of the relevant passages | Relevant to claim No. |
|---|---|---|
| A | CN 101357951 A (NANFANG LEE KUM KEE CO., LTD. et al.) 04 February 2009 (2009-02-04)<br>entire document | 1-10 |
| A | CN 111533823 A (GUANGZHOU YOROYAL BIOLOGICAL TECHNOLOGY CO., LTD.) 14 August 2020 (2020-08-14)<br>entire document | 1-10 |
| A | CN 102617745 A (GUANGDONG INSTITUTE OF MICROBIOLOGY et al.) 01 August 2012 (2012-08-01)<br>entire document | 1-10 |
| A | CN 101367881 A (NANJING AGRICULTURAL UNIVERSITY) 18 February 2009 (2009-02-18)<br>entire document | 1-10 |
| A | WO 2022062380 A1 (GUANGDONG INSTITUTE OF MICROBIOLOGY (GUANGDONG DETECTION CENTER OF MICROBIOLOGY) et al.) 31 March 2022 (2022-03-31)<br>entire document | 1-10 |

Form PCT/ISA/210 (second sheet) (July 2022)

**INTERNATIONAL SEARCH REPORT**
**Information on patent family members**

International application No.
**PCT/CN2024/080445**

| Patent document cited in search report | | | Publication date (day/month/year) | Patent family member(s) | | | Publication date (day/month/year) |
|---|---|---|---|---|---|---|---|
| CN | 108976314 | A | 11 December 2018 | None | | | |
| CN | 108250320 | A | 06 July 2018 | CN | 108250320 | B | 21 April 2020 |
| CN | 107857828 | A | 30 March 2018 | None | | | |
| CN | 101357951 | A | 04 February 2009 | None | | | |
| CN | 111533823 | A | 14 August 2020 | None | | | |
| CN | 102617745 | A | 01 August 2012 | CN | 102617745 | B | 13 November 2013 |
| CN | 101367881 | A | 18 February 2009 | CN | 101367881 | B | 08 December 2010 |
| WO | 2022062380 | A1 | 31 March 2022 | CN | 112094358 | A | 18 December 2020 |
| | | | | CN | 112094358 | B | 27 May 2022 |

Form PCT/ISA/210 (patent family annex) (July 2022)